(19) Europäisches Patentamt
European Patent Office
Office européen des brevets

(11) EP 0 714 892 B1

(12) EUROPEAN PATENT SPECIFICATION

(45) Date of publication and mention of the grant of the patent:
**07.01.2004 Bulletin 2004/02**

(21) Application number: **95116130.6**

(22) Date of filing: **12.10.1995**

(51) Int Cl.[7]: **C07D 249/08**, C07D 487/04, C07F 9/6518, G03C 7/38 // (C07D487/04, 249:00, 209:00)

(54) **1H-pyrrolo [1, 2-b][1, 2, 4] Triazole derivatives and their preparation via 1H-1,2,4-triazole derivatives**

1H-Pyrrolo [1,2-b][1,2,4] triazol-Derivate und deren Herstellung über 1H-1,2,4-triazol-Derivate

Dérivés de 1H-Pyrrolo[1,2-b][1,2,4]triazole et leur préparation via dérivés de 1H-1,2,4-triazole

(84) Designated Contracting States:
**CH DE GB LI NL**

(30) Priority: **12.10.1994 JP 27186994**

(43) Date of publication of application:
**05.06.1996 Bulletin 1996/23**

(73) Proprietor: **FUJI PHOTO FILM CO., LTD.**
**Kanagawa-ken (JP)**

(72) Inventors:
- **Ito, Takayuki, c/o Fuji Photo Film Co., Ltd. Minami-Ashigara-shi, Kanagawa (JP)**
- **Shimada, Yasuhiro, c/o Fuji Photo Film Co., Ltd. Minami-Ashigara-shi, Kanagawa (JP)**
- **Matsuoka, Koushin, c/o Fuji Photo Film Co., Ltd. Minami-Ashigara-shi, Kanagawa (JP)**

(74) Representative: **Grünecker, Kinkeldey,**
**Stockmair & Schwanhäusser Anwaltssozietät**
**Maximilianstrasse 58**
**80538 München (DE)**

(56) References cited:

| | |
|---|---|
| **EP-A- 0 491 197** | **EP-A- 0 518 238** |
| **EP-A- 0 545 300** | **EP-A- 0 566 115** |
| **EP-A- 0 628 867** | **JP-A- 5 333 503** |

- **CHEMICAL ABSTRACTS, vol. 123, no. 5, 31 July 1995 Columbus, Ohio, US; abstract no. 55892, ITO T. 'Method for preparation of 1H-pyrrolo[1,2-b][1,2,4]triazole derivative' & JP-A-07 048 376 (FUJI PHOTO FILM CO. LTD.) 21 February 1995**
- **CHEMICAL REVIEWS, vol. 91, no. 2, March 1991 pages 165-195, HANSCH C. ET AL. 'A survey of Hammett substituent constants and resonance and field parameters'**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

FIELD OF THE INVENTION

The present invention relates to a novel 1H-pyrrolo[1,2-b][1,2,4]triazole derivative useful as an intermediate for physiologically active substances such as medicines and agricultural chemicals or as a precursor for photographic cyan couplers, dyes for dye-providing heat transfer materials, and filter dyes for use in solid camera tubes or liquid-crystal color displays. This invention also relates to a 1H-1,2,4-triazole derivative which serves as an important intermediate for efficiently synthesizing the 1H-pyrrolo[1,2-b][1,2,4]triazole derivative.

BACKGROUND OF THE INVENTION

The reactivity of 1H-pyrrolo[1,2-b][1,2,4]triazole derivatives is discussed in *Ukrainskii Khimicheskii Zhurnal*, Vol. 41, No. 2, pp. 181-185 (1975) and *Khimiya Geterotsiklicheskikh Scedinenii*, No. 2, pp. 261-267 (Feb., 1974). These 1H-pyrrolo[1,2-b][1,2,4]triazole derivatives are known as a medicine or the like as disclosed in U.S. Patents 4,358,457 and 4,962,202. The 1H-pyrrolo[1,2-b][1,2,4]triazole derivatives are also known as a photographic magenta coupler or magenta dye as disclosed in Shōwa 60-nendo Nenji Taikai Kōenyōshi-shū (Abstract Papers for Annual Meeting of Photographic Science and Technology Society of Japan, 1985), JP-A-62-278552, JP-A-62-279339, JP-A-1-288835, U.S. Patent 4,910,127, and European Patent EP 0,491,197 A1. (The term "JP-A" as used herein means an "unexamined published Japanese patent application.")
In European Patent EP 0,491,197 A1, JP-A-5-313324, JP-A-5-202049, JP-A-5-232649, JP-A-5-323536, JP-A-6-172357, etc. is disclosed that 1H-pyrrolo[1,2-b][1,2,4]triazole derivatives can be converted to compounds useful as a photographic cyan coupler by incorporating electron-withdrawing groups thereinto at the 6- and 7-positions. Processes for synthesizing such 1H-pyrrolo[1,2-b][1,2,4]triazole derivatives having electron-withdrawing groups at the 6- and 7-positions are described in, e.g., JP-A-5-202004 and JP-A-5-255333, in which processes a 1H-1,2,4-triazole derivative represented by the following general formula (IV) is used as a starting material.

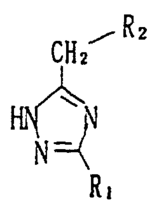

(IV)

In the above formula, $R_1$ and $R_2$ have the same meanings as in formula (I) for use in the present invention.
The first step in each of these processes comprises alkylating the 1-position nitrogen atom of the 1H-1,2,4-triazole derivative represented by general formula (IV). However, the selectivity of the alkylation is not always satisfactory, so that the 2-position nitrogen atom is also alkylated disadvantageously depending on the kind of the substituent $R_1$. Moreover, the reaction for forming a pyrrole ring after the alkylation of the 1-position nitrogen atom does not always proceed in a high yield depending on the kinds of the substituents $R_1$ and $R_2$. The prior art processes have a further drawback as follows. The 1H-pyrrolo[1,2-b][1,2,4]triazole derivatives that can be synthesized by these processes are limited to those which are unsubstituted at the 5-position (four-equivalent couplers). The 5-position unsubstituted 1H-pyrrolo[1,2-b][1,2,4]triazole derivatives thus obtained can be converted to two-equivalent couplers by incorporating a halogen atom at the 5-position by means of electrophilic substitution reaction. However, the resulting triazole derivatives have problems that the 1-position proton has a considerably reduced $pK_a$ and these derivatives have impaired thermal stability. In addition, it has been exceedingly difficult in the above-described processes to incorporate an oxygenic functional group such as an acyloxy group at the 5-position.
European Patent EP 0,566,115 A1 discloses a silver halide color photographic material comprising a cyan coupler represented by the formula

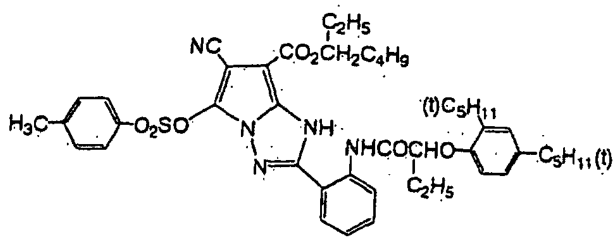

or

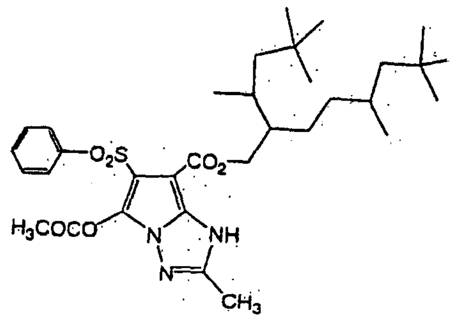

U.S. Patent 5,415,982 discloses a silver halide color photographic material comprising a cyan coupler represented by the formula

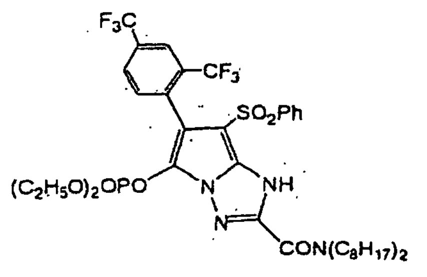

or

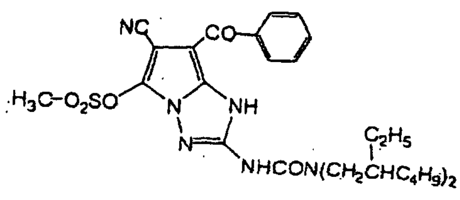

SUMMARY OF THE INVENTION

An object of the present invention is to provide an intermediate which is necessary for synthesizing a 1H-pyrrolo[1,2-b][1,2,4]triazole derivative in a high yield through a novel route in which a 1H-1,2,4-triazole derivative represented by general formula (IV) is used as a starting material but is not subjected to an N-alkylation reaction as the first step.

Another object of the present invention is to provide a 1H-pyrrolo[1,2-b][1,2,4]triazole derivative having an oxygenic functional group at the 5-position which derivative has a higher $pK_a$ than 5-halogen-substituted corresponding compounds and is thermally stable.

As a result of intensive studies made in order to accomplish the above objects, it has become possible to synthesize in a high yield a 1H-pyrrolo[1,2-b][1,2,4]triazole derivative represented by formula (III) by using 1H-1,2,4-triazole derivatives respectively represented by the following formulae (I) and (II). It has been ascertained that the thus-obtained 1H-pyrrolo[1,2-b][1,2,4]triazole derivative represented by formula (III) has a $pK_a$ value higher by about 1 than those of 5-halogen-substituted corresponding compounds and also has higher thermal stability than the corresponding compounds.

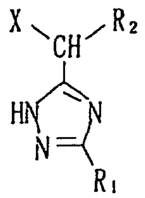

(I)

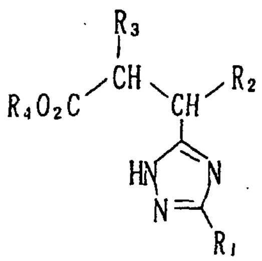

(II)

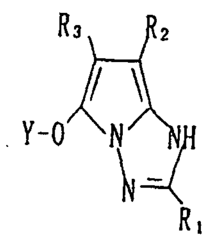

(III)

In the above formulae, $R_1$ represents an optionally substituted aliphatic group or an optionally substituted aryl group. $R_2$ and $R_3$ each represents an electron-withdrawing group having a Hammett's substituent constant $\sigma_p$ of from 0.2 to 1.0. $R_4$ represents a hydrogen atom, an aliphatic group, or an aryl group. X represents a halogen atom. Y represents -C(=O)-N(Ra')Ra and wherein

Ra and Ra' each represents a hydrogen atom, an aliphatic group which may be substituted, or an aryl group which may be substituted, and Ra and Ra' may be bonded to each other to form a ring .

The present invention provides 1H-1,2,4-triazole derivatives according to claims 1 and 8, and a process for the preparation of 1H-1,2,4-triazole derivatives according to claim 16. The present invention further provides 1H-pyrrolo[1,2-b][1,2,4]triazole derivatives according to claim 17, and processes for the preparation of 1H-pyrrolo[1,2-b][1,2,4]triazole derivatives according to claims 24 and 25. Preferred embodiments of the present invention are set forth in the sub-claims.

DETAILED DESCRIPTION OF THE INVENTION

The compounds respectively represented by formulae (I), (II), and (III) are explained below in detail.

$R_1$ represents an optionally substituted aliphatic group or an optionally substituted aryl group.

The aliphatic group represented by $R_1$ is a linear or branched alkyl group, an aralkyl group, an alkenyl group,

an alkynyl group, a cycloalkyl group or a cycloalkenyl group, each having up to 36 carbon atoms. Examples thereof include methyl, ethyl, propyl, isopropyl, t-butyl, tridecyl, t-amyl, t-octyl, octadecyl, oleyl, propargyl, cyclohexyl, cyclopentyl, and 4-butylcyclohexyl.

This aliphatic group may be substituted with a substituent. Examples of preferred substituents include halogen atoms (e.g., fluorine atom, chlorine atom, bromine atom, and iodine atom), a cyano group, a nitro group, aryl groups (e.g., phenyl and naphthyl), alkoxy groups (e.g., methoxy, ethoxy, and n-butoxy), aryloxy groups (e.g., phenoxy and naphthyloxy), alkylthio groups (e.g., methylthio and n-propylthio), arylthio groups (e.g., phenylthio), alkylsulfonyl groups (e.g., methanesulfonyl and ethanesulfonyl), arylsulfonyl groups (e.g., benzenesulfonyl), a formyl group, acyl groups (e.g., acetyl, benzoyl, and propionyl), acyloxy groups (e.g., acetyloxy and benzoyloxy), alkoxycarbonyl groups (e.g., methoxycarbonyl, butoxycarbonyl, and n-octyloxycarbonyl), aryloxycarbonyl groups (e.g., phenoxycarbonyl), carbonamido groups (e.g., acetylamino, propionylamino, and benzoylamino), sulfonamido groups (e.g., methanesulfonylamido, ethanesulfonylamido, and benzenesulfonylamido), carbamoyl groups (e.g., dimethylaminocarbonyl, ethylaminocarbonyl, and dioctylaminocarbonyl), sulfamoyl groups (e.g., methylaminosulfonyl, diethylaminosulfonyl, and n-butylaminosulfonyl), ureido groups (e.g., dimethylaminocarbonylamino and anilinocarbonylamino), alkoxycarbonylamino groups (e.g., methoxycarbonylamino and isobutoxycarbonylamino), amino groups (e.g., amino, diethylamino, and dihexylamino), imido groups (e.g., phthalimido), and heterocyclic groups (e.g., furyl, thienyl, and morpholino). These substituents may be further substituted, if possible, with any of these substituents.

The aryl group represented by $R_1$ is an aryl group having from 6 to 36 carbon atoms. Examples thereof include phenyl, 1-naphthyl, and 2-naphthyl.

This aryl group may be substituted with a substituent. Examples of preferred substituents include halogen atoms (e.g., fluorine atom, chlorine atom, bromine atom, and iodine atom), a cyano group, a nitro group, aliphatic groups such as those enumerated above, aryl groups (e.g., phenyl and naphthyl), alkoxy groups (e.g., methoxy, ethoxy, and n-butoxy), aryloxy groups (e.g., phenoxy and naphthyloxy), alkylthio groups (e.g., methylthio and n-propylthio), arylthio groups (e.g., phenylthio), alkylsulfonyl groups (e.g., methanesulfonyl and ethanesulfonyl), arylsulfonyl groups (e.g., benzenesulfonyl), a formyl group, acyl groups (e.g., acetyl, benzoyl, and propionyl), acyloxy groups (e.g., acetyloxy and benzoyloxy), alkoxycarbonyl groups (e.g., methoxycarbonyl, butoxycarbonyl, and n-octyloxycarbonyl), aryloxycarbonyl groups (e.g., phenoxycarbonyl), carbonamido groups (e.g., acetylamino, propionylamino, and benzoylamino), sulfonamido groups (e.g., methanesulfonylamido, ethanesulfonylamido, and benzenesulfonylamido), carbamoyl groups (e.g., dimethylaminocarbonyl, ethylaminocarbonyl, and dioctylaminocarbonyl), sulfamoyl groups (e.g., methylaminosulfonyl, diethylaminosulfonyl, and n-butylaminosulfonyl), ureido groups (e.g., dimethylaminocarbonylamino and anilinocarbonylamino), alkoxycarbonylamino groups (e.g., methoxycarbonylamino and isobutoxycarbonylamino), amino groups (e.g., amino, diethylamino, and dihexylamino), imido groups (e.g., phthalimido), and heterocyclic groups (e.g., furyl, thienyl, and morpholino). These substituents may be further substituted, if possible, with any of these substituents.

$R_2$ and $R_3$ each represents an electron-withdrawing group having a Hammett's substituent constant $\sigma_p$ (hereinafter referred to simply as "$\sigma_p$ value") of from 0.20 to 1.0, preferably from 0.30 to 0.8. The Hammett's rule is the empirical rule proposed by L.P. Hammett in 1935 in order to quantitatively discuss the influence of substituents on the reaction of benzene derivatives or on the equilibrium thereof. At present, this rule is generally regarded as valid. The substituent constants determined by the Hammett's rule include $\sigma_p$ value and $\sigma_m$ value, which are described in many widespread books. For example, detailed descriptions are given in J.A. Dean "Lange's Handbook of Chemistry" 12th edition, 1979 (Mc Graw-Hill); "Kagaku No Ryōiki, Zōkan (The Domain of Chemistry, Extra Edition)," No. 122, pp. 96-103, 1979 (Nankōdō); and *Chemical Reviews*, Vol. 91, pp. 165-195, 1991. Although $R_2$ and $R_3$ in this invention have a Hammett's substituent constant $\sigma_p$ within the above-specified range, these groups should not be construed as being limited to substituents selected from those whose $\sigma_p$ values are known from these books. Namely, any substituent whose $\sigma_p$ value has been unknown and is not given in the literature can, of course, be included in the substituents of $R_2$.and $R_3$, as long as the $\sigma_p$ value thereof as measured according to the Hammett's rule is within that range.

Examples of $R_2$ and $R_3$, which each is an electron-withdrawing group having a $\sigma_p$ value of from 0.20 to 1.0, include acyl groups, acyloxy groups, a carbamoyl group, aliphatic-oxycarbonyl groups, aryloxycarbonyl groups, a cyano group, a nitro group, dialkylphosphono groups, diarylphosphono groups, diarylphosphinyl groups, alkylsulfinyl groups, arylsulfinyl groups, alkylsulfonyl groups, arylsulfonyl groups, sulfonyloxy groups, acylthio groups, a sulfamoyl group, a thiocyanate group, a thiocarbonyl group, alkyl groups each substituted with at least two halogen atoms, alkoxy groups each substituted with at least two halogen atoms, aryloxy groups each substituted with at least two halogen atoms, alkylamino groups each substituted with at least two halogen atoms, alkylthio groups each substituted with at least two halogen atoms, aryl groups substituted with another electron-withdrawing group having a $\sigma_p$ value of 0.20 or higher, heterocyclic groups, a chlorine atom, a bromine atom, an azo group, and a selenocyanate group. These substituents may be further substituted, if possible, with a substituent such as those enumerated above as examples of substituents which the aliphatic or aryl group represented by $R_1$ may have.

Representative examples of the electron-withdrawing group having a $\sigma_p$ value of from 0.2 to 1.0 are given

below together with their $\sigma_p$ values: bromine atom (0.23), chlorine atom (0.23), cyano group (0.66), nitro group (0.78), trifluoromethyl group (0.54), tribromomethyl group (0.29), trichloromethyl group (0.33), carboxyl group (0.45), acetyl group (0.50), benzoyl group (0.43), acetyloxy group (0.31), trifluoromethanesulfonyl group (0.92), methanesulfonyl group (0.72), benzenesulfonyl group (0.70), methanesulfinyl group (0.49), carbamoyl group (0.36), methoxycarbonyl group (0.45), ethoxycarbonyl group (0.45), phenoxycarbonyl group (0.44), pyrazolyl group (0.37), methanesulfonyloxy group (0.36), dimethoxyphosphoryl group (0.60), and sulfamoyl group (0.57).

Examples of desirable substituents for $R_2$ include aliphatic-oxycarbonyl groups (a linear or branched alkoxycarbonyl group, an aralkyloxycarbonyl group, an alkenyloxycarbonyl group, an alkynyloxycarbonyl group, a cycloalkoxycarbonyl group, or a cycloalkenyloxycarbonyl group, each having up to 36 carbon atoms, e.g., methoxycarbonyl, ethoxycarbonyl, dodecyloxycarbonyl, octadecyloxycarbonyl, 2-ethylhexyloxycarbonyl, sec-butyloxycarbonyl, oleyloxycarbonyl, benzyloxycarbonyl, propargyloxycarbonyl, cyclopentyloxycarbonyl, cyclohexyloxycarbonyl, and 2,6-di-t-butyl-4-methylcyclohexyloxycarbonyl), carbamoyl groups (carbamoyl groups each having from 1 to 36 carbon atoms, e.g., diethylcarbamoyl and dioctylcarbamoyl), sulfamoyl groups (sulfamoyl groups each having from 1 to 36 carbon atoms, e.g., dimethylsulfamoyl and dibutylsulfamoyl), dialkylphosphono groups (dialkylphosphono groups each having from 2 to 36 carbon atoms, e.g., diethylphosphono and dioctylphosphono), and diarylphosphono groups (diarylphosphono groups each having from 12 to 50 carbon atoms, e.g., diphenylphosphono and di(p-toluyl)phosphono). Preferred substituents for $R_2$ are aliphatic-oxycarbonyl groups, with the aliphatic-oxycarbonyl groups represented by the following formula (V) being especially preferred.

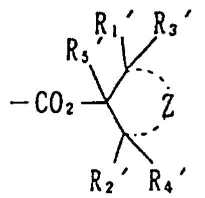

(V)

In the above formula, $R_1$' and $R_2$' each represents an aliphatic group (e.g., a linear or branched alkyl group, an aralkyl group, an alkenyl group, an alkynyl group, a cycloalkyl group or a cycloalkenyl group, each having up to 36 carbon atoms). Specific examples of the aliphatic group include methyl, ethyl, propyl, isopropyl, t-butyl, t-amyl, t-octyl, tridecyl, cyclopentyl, and cyclohexyl.

$R_3$', $R_4$', and $R_5$' each represents a hydrogen atom or an aliphatic group. Examples of the aliphatic group include the groups enumerated above with regard to $R_1$' and $R_2$'. Preferably, $R_3$', $R_4$', and $R_5$' each is a hydrogen atom.

Z represents a group of non-metallic atoms necessary for forming a 5- to 8-membered ring. Desirable nonmetallic atoms include nitrogen atom, oxygen atom, sulfur atom, and carbon atom, with carbon atom being preferred.

Examples of the ring containing Z as a constituent include a cyclopentane ring, a cyclohexane ring, a cycloheptane ring, a cyclooctane ring, a cyclohexene ring, a piperazine ring, an oxane ring, and a thiane ring. These rings may be substituted with a substituent such as those enumerated above with regard to $R_1$. The ring containing Z as a constituent is desirably an optionally substituted cyclohexane ring, preferably a cyclohexane ring substituted at the 4-position with an alkyl group having from 1 to 24 carbon atoms (the alkyl group may be substituted with a substituent such as those enumerated above with regard to $R_1$).

Examples of desirable substituents for $R_3$ include a cyano group, aliphatic-oxycarbonyl groups (a linear or branched alkoxycarbonyl group, an aralkyloxycarbonyl group, an alkenyloxycarbonyl group, an alkynyloxycarbonyl group, a cycloalkoxycarbonyl group, or a cycloalkenyloxycarbonyl group, each having up to 36 carbon atoms; e.g., methoxycarbonyl, ethoxycarbonyl, dodecyloxycarbonyl, octadecyloxycarbonyl, 2-ethylhexyloxycarbonyl, sec-butyloxycarbonyl, oleyloxycarbonyl, benzyloxycarbonyl, propargyloxycarbonyl, cyclopentyloxycarbonyl, cyclohexyloxycarbonyl, and 2,6-di-t-butyl-4-methylcyclohexyloxycarbonyl), dialkylphosphono groups (dialkylphosphono groups each having from 2 to 36 carbon atoms, e.g., diethylphosphono and dimethylphosphono), alkyl- or arylsulfonyl groups (alkyl- or arylsulfonyl groups each having up to 36 carbon atoms, e.g., methanesulfonyl, butanesulfonyl, benzenesulfonyl, and p-toluenesulfonyl), and fluoroalkyl groups (fluoroalkyl groups each having from 1 to 36 carbon atoms, e.g., trifluoromethyl). Preferred substituents for $R_3$ are cyano group, aliphatic-oxycarbonyl groups, and fluoroalkyl groups, with cyano group being the most desirable.

$R_4$ represents a hydrogen atom, an aliphatic group, or an aryl group.

The aliphatic group and aryl group represented by $R_4$ respectively have the same meanings as the aliphatic group and aryl group described hereinabove with regard to $R_1$. Examples of $R_4$ include a hydrogen atom, methyl, ethyl, propyl, butyl, and phenyl. $R_4$ is desirably a hydrogen atom or an alkyl group. The alkyl group is preferably an alkyl

group having from 1 to 7 carbon atoms, especially preferably methyl or ethyl.

X represents a halogen atom, desirably a bromine atom or a chlorine atom, preferably a bromine atom.

Y represents -C(=O)-N(Ra')Ra and wherein Ra and Ra' each represents a hydrogen atom, an aliphatic group which may be substituted, or an aryl group which may be substituted, and Ra and Ra' may be bonded to each other to form a ring.

The aliphatic group and aryl group represented by Ra and Ra' respectively have the same meanings as the aliphatic group and aryl group described hereinabove with regard to $R_1$. Ra and Ra' may be the same or different, and may be bonded to each other to form a ring.

In the case where Ra and Ra' are bonded to each other to form a ring, examples of the cyclic compound include cyclic amide compounds, cyclic imide compounds, cyclic urea compounds, and cyclic amines such as imidazole, pyrazole, triazole, lactam compounds, piperidine, pyrrolidine, pyrrole, morpholine, pyrazolidine, and pyrazoline.

Especially preferred embodiments of the 1H-1,2,4-triazole derivative and 1H-pyrrolo[1,2-b][1,2,4]triazole derivative of the present invention are represented by the following general formulae (I'), (II'), and (III').

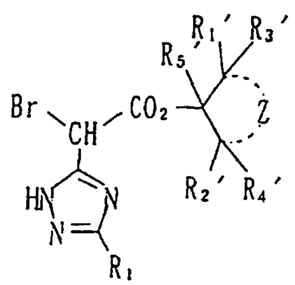

(I')

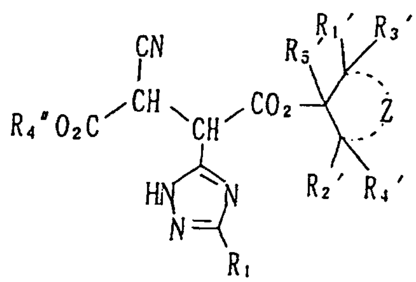

(II')

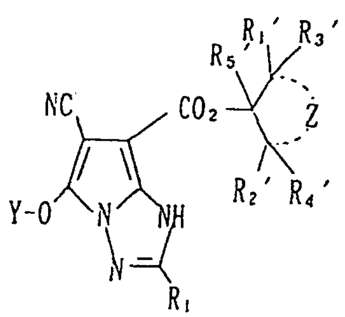

(III')

In the above formulae, $R_1$, $R_1$' to $R_5$', Y, and Z have the same meanings as defined hereinabove, and $R_4$" represents a hydrogen atom or an alkyl group having from 1 to 7 carbon atoms. $R_1$ preferably represents a branched alkyl group or a cycloalkyl group . $R_3$', $R_4$', and $R_5$' each preferably represents a hydrogen atom. $R_4$" preferably represents a hydrogen atom, methyl, or ethyl, and the ring represented by Z is preferably a cyclohexane ring.

Specific examples of the compounds respectively represented by formulae (I), (II), and (III) are given below.

(I-1)

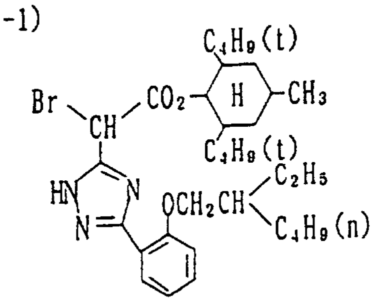

(I-2)

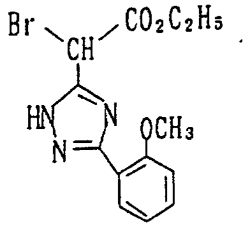

(I-3)

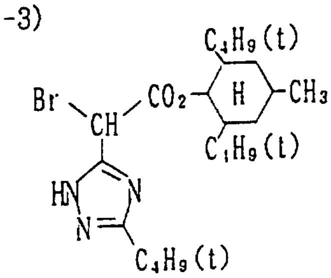

(I-4)

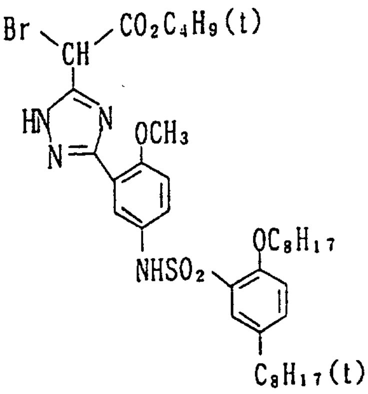

(I-5)

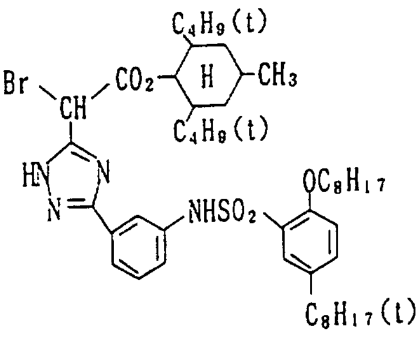

(I-6)

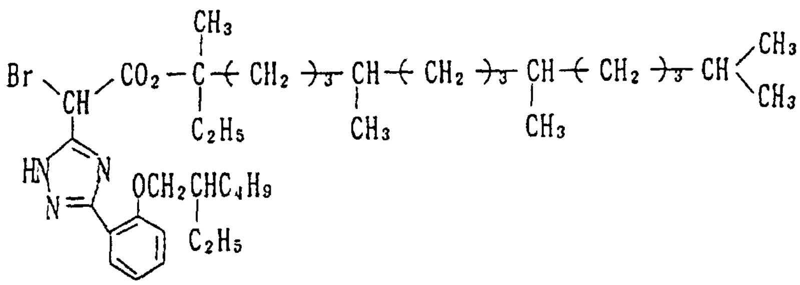

(I-7)

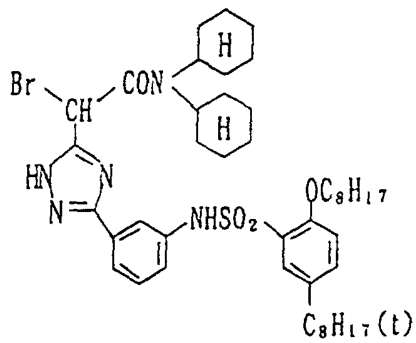

(I-8)

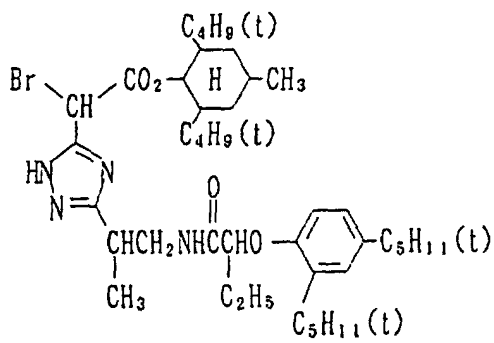

(I-9)

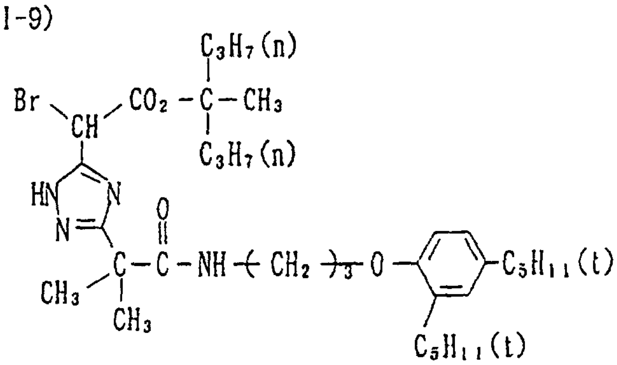

(I-10)

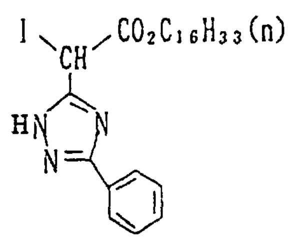

(I-11)

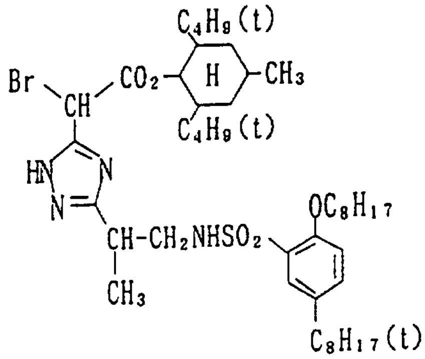

(I-12)

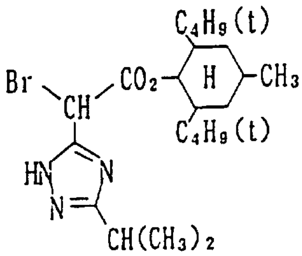

(I-13)

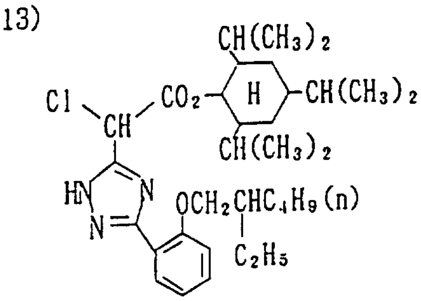

(I-14)

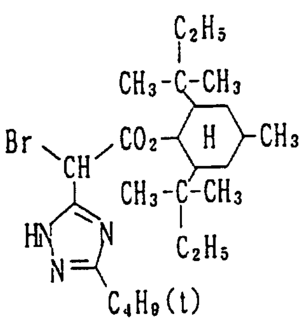

(I-15)

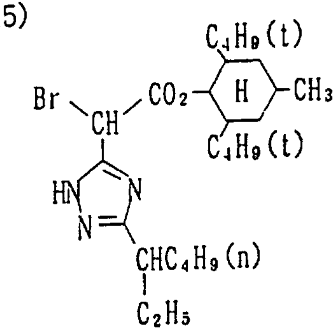

(I-16)

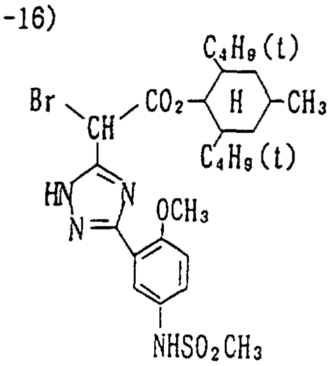

(I-17)

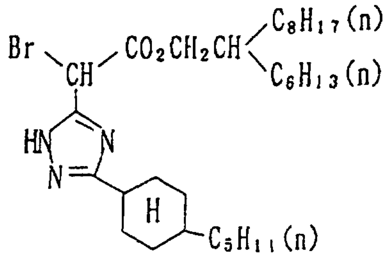

(I-18)

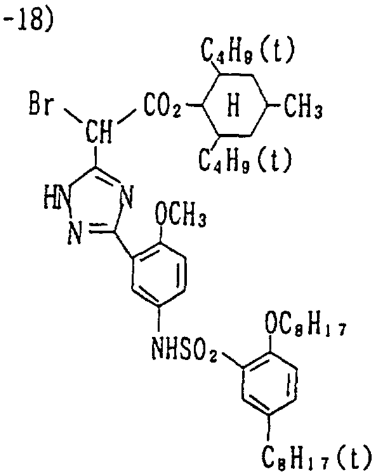

(I-19)

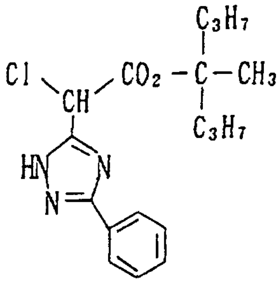

(I-20)

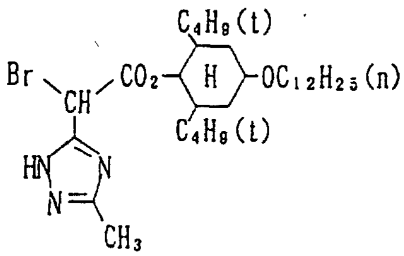

(I-21)

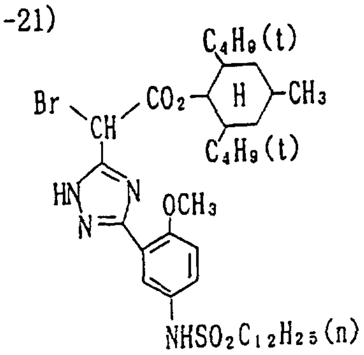

(I-22)

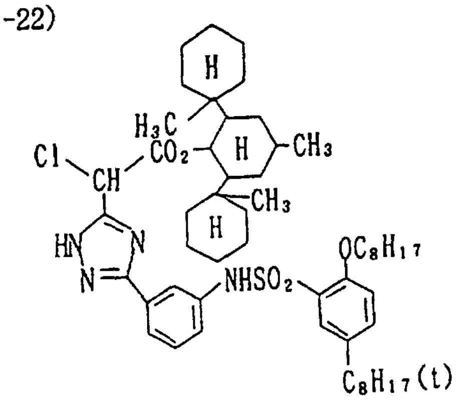

(I-23)

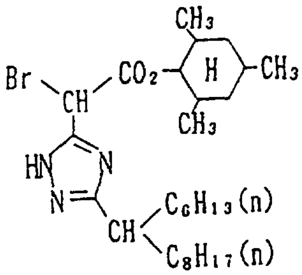

(I-24)

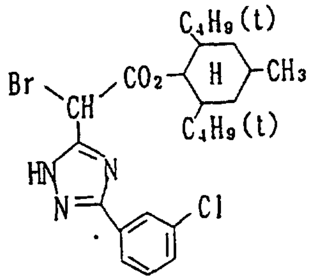

(I-25)

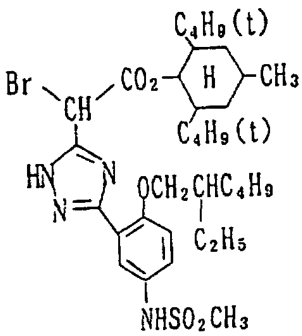

(I-26)

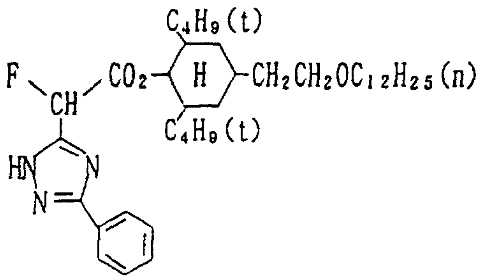

(I-27)

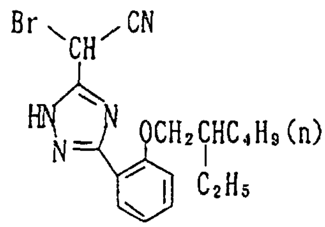

(I-28)

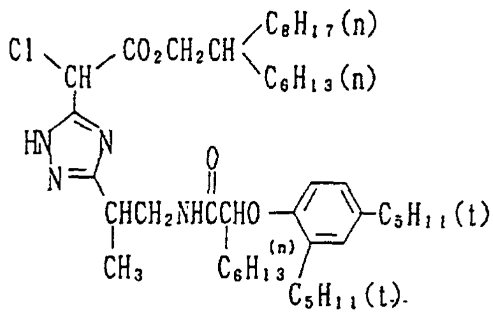

(I-29)

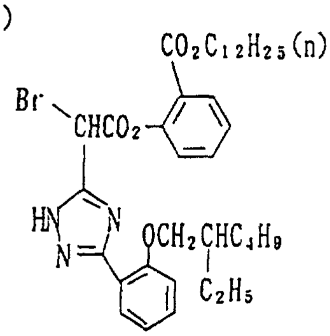

(I-30)

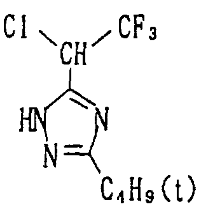

(I-31)

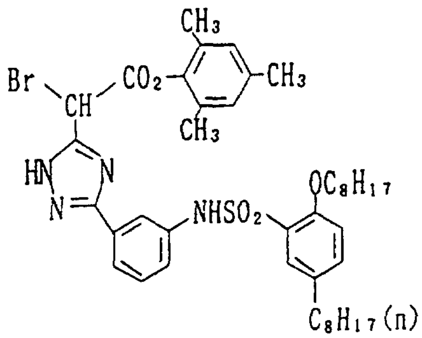

(I-32)

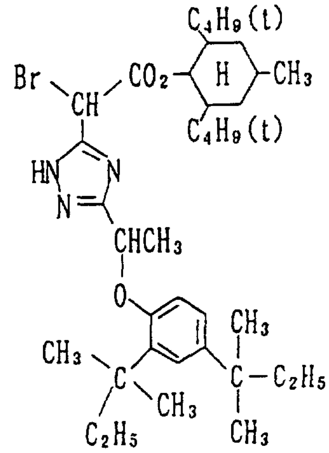

(I-33)

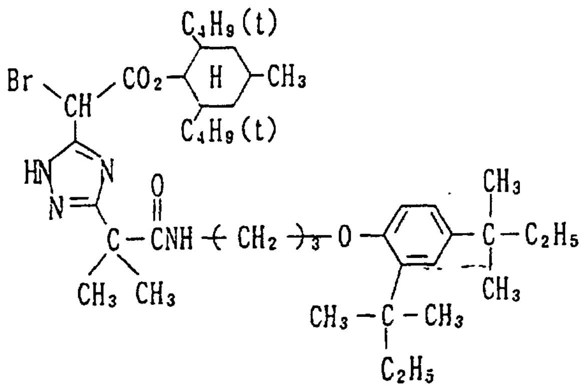

(I-34)

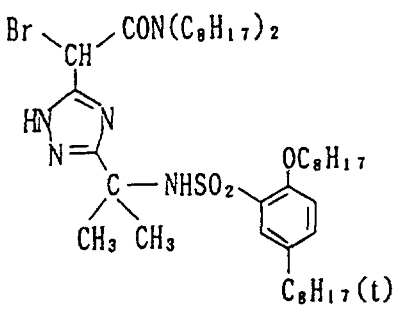

(I-35)

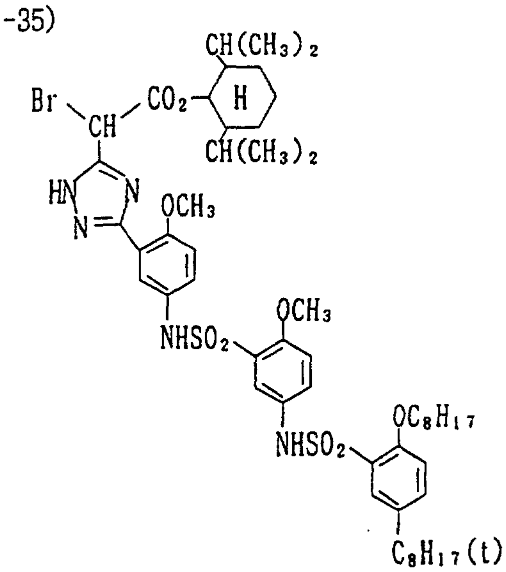

(I-36)

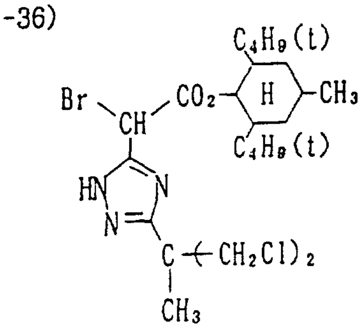

(I-37)

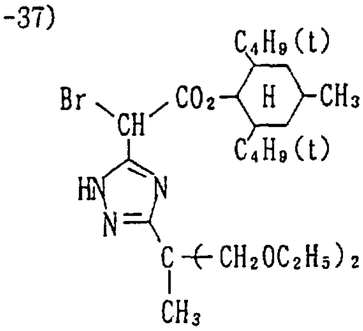

(I-38)

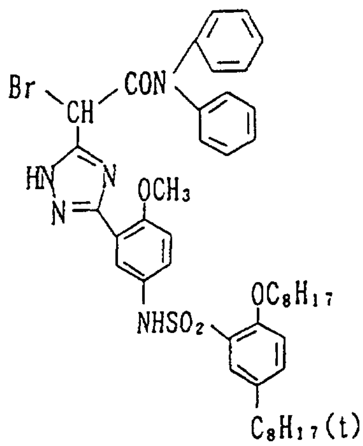

(I-39)

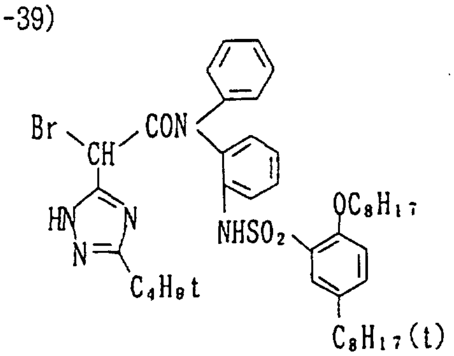

(I-40)

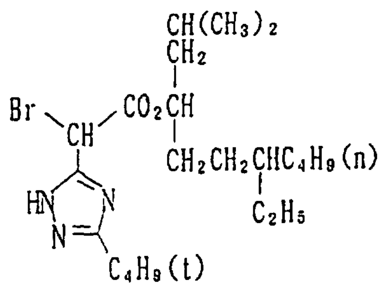

(II-1)

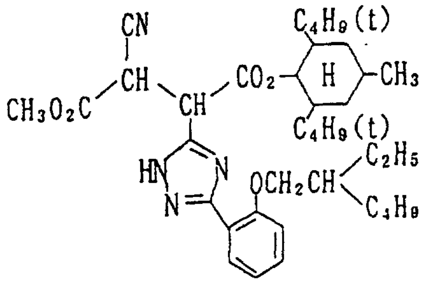

(II-2)

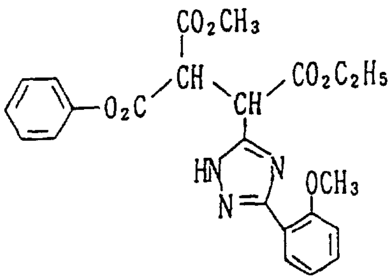

(II-3)

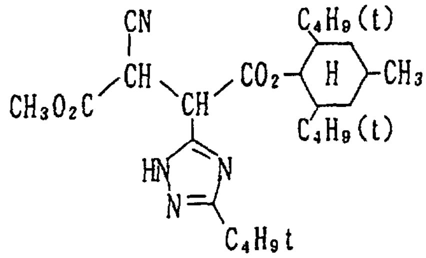

(II-4)

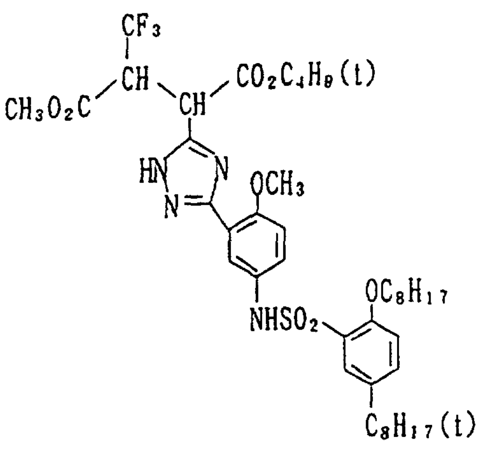

(II-5)

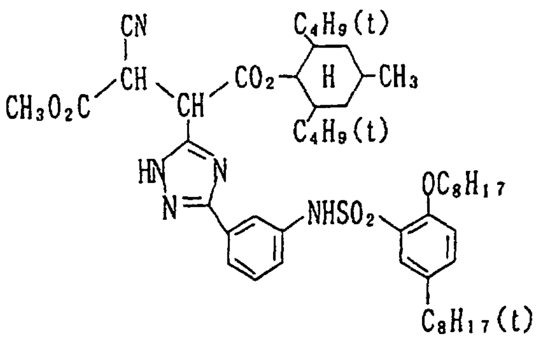

(II-6)

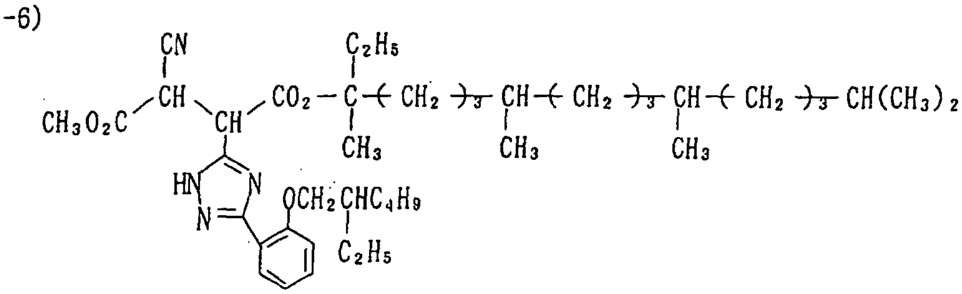

(II-7)

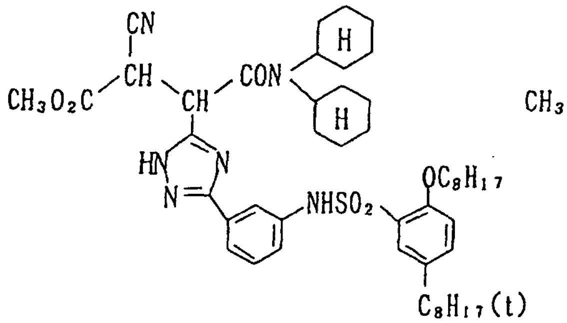

(II-8)

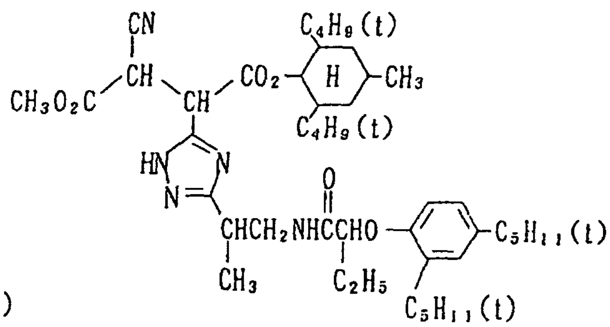

(II-9)

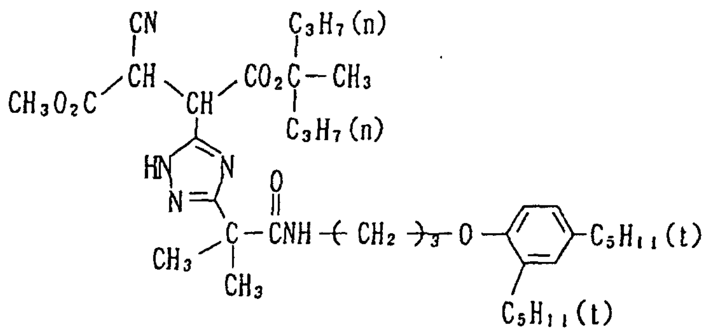

(II-10)

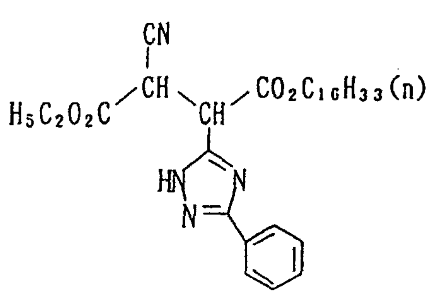

(II-11)

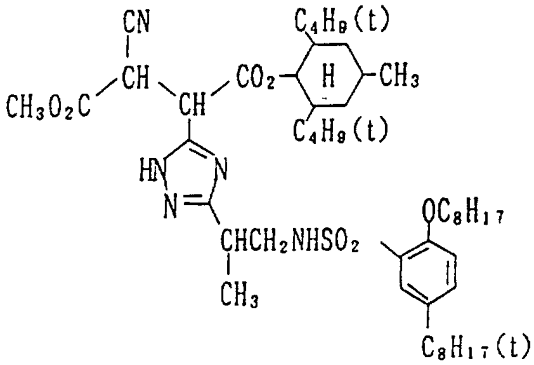

(II-12)

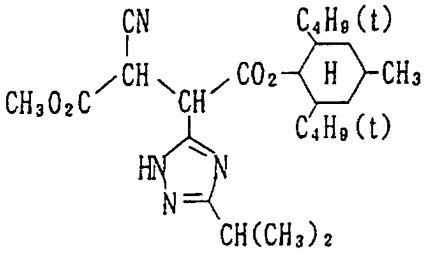

(II-13)

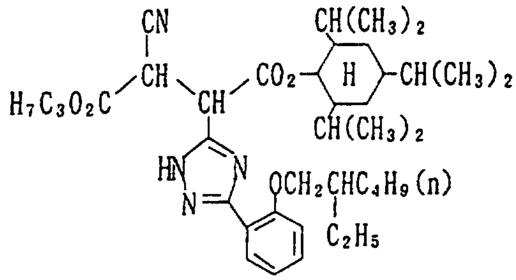

(II-14)

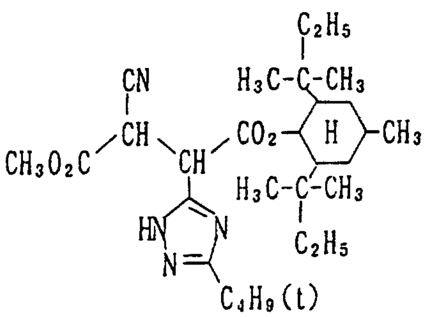

(II-15)

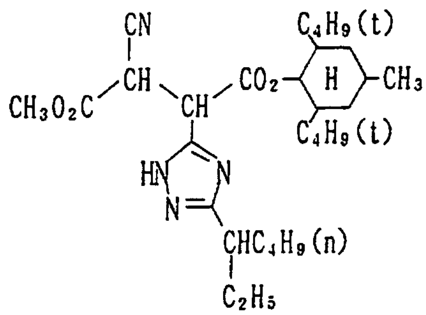

(II-16)

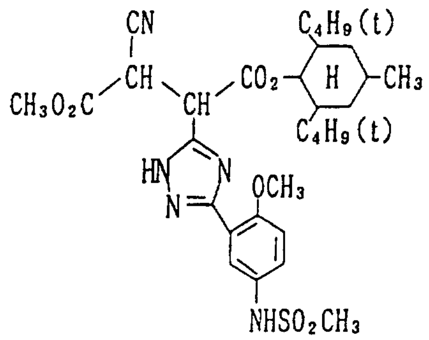

(II-17)

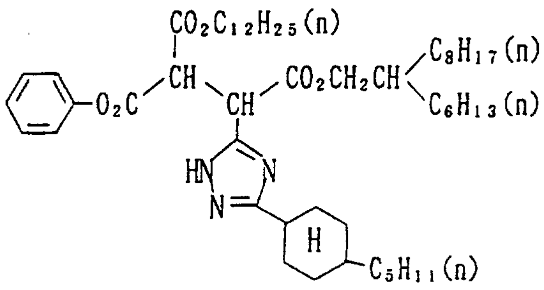

(II-18)

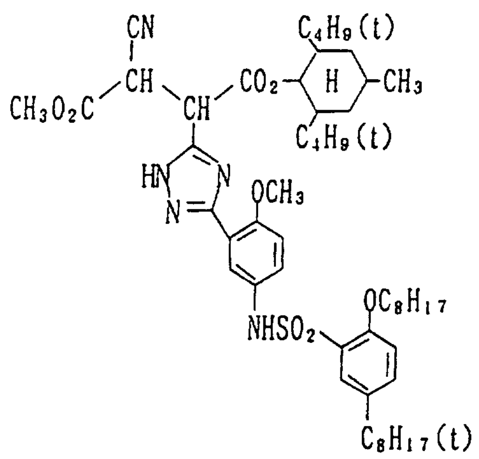

(II-19)

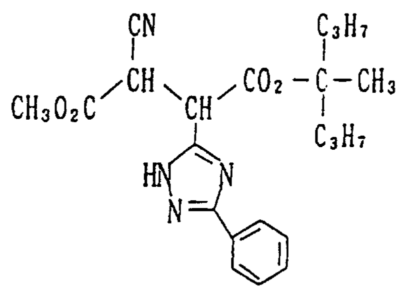

(II-20)

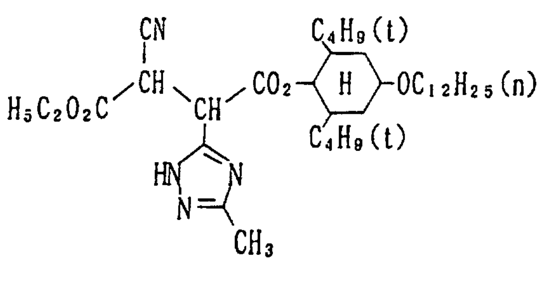

(II-21)

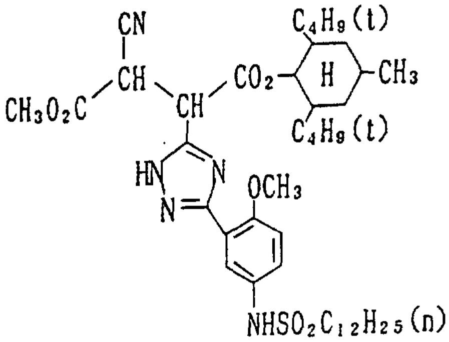

(II-22)

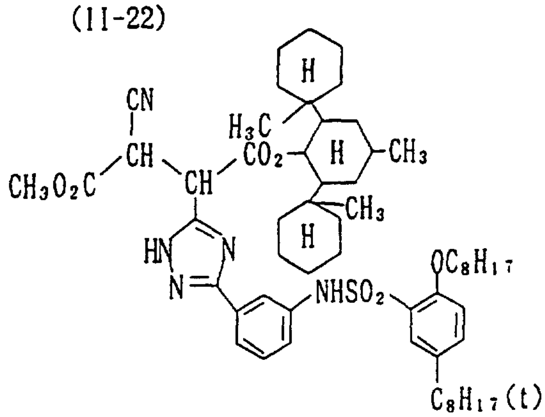

(II-23)

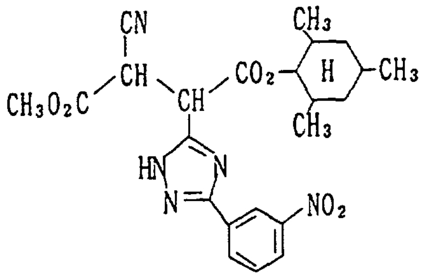

(II-24)

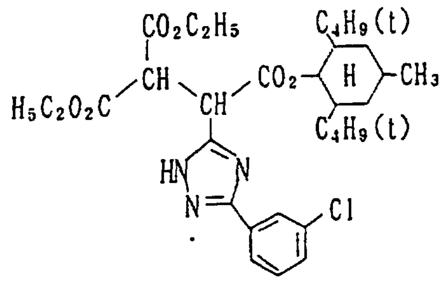

(II-25)

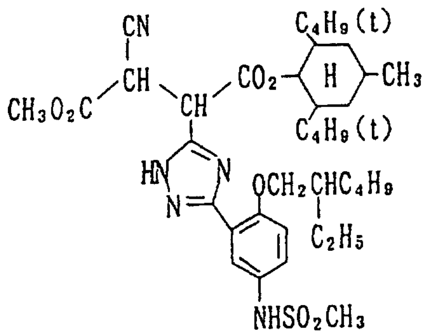

(II-26)

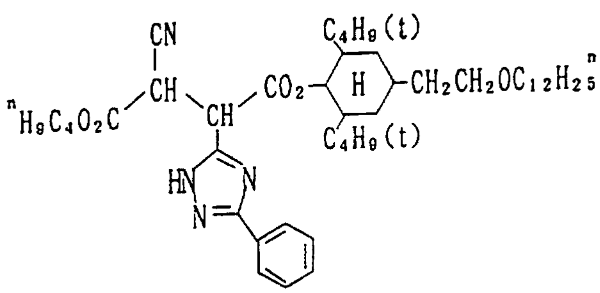

(II-27)

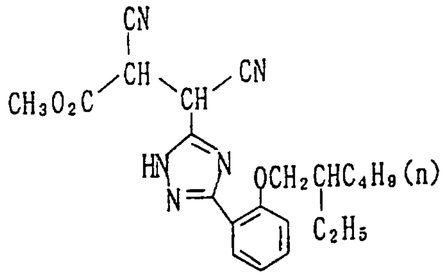

(II-28)

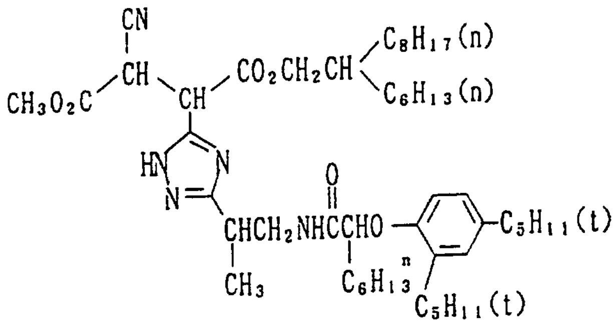

(II-29)

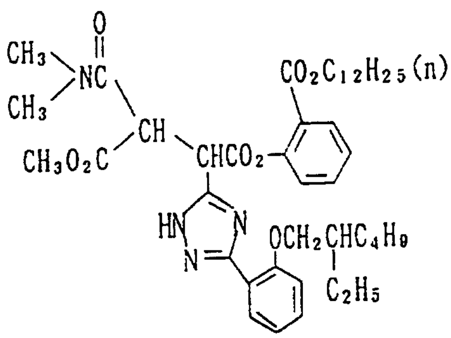

(II-30)

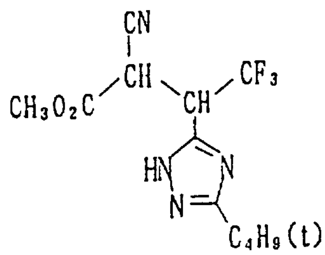

(II-31)

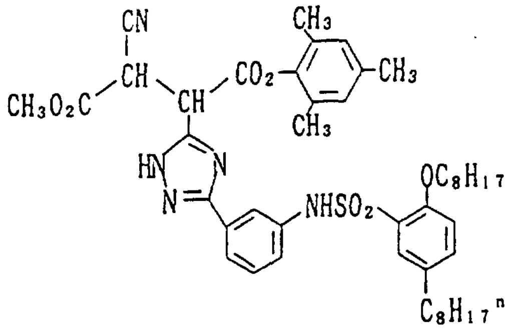

(II-32)

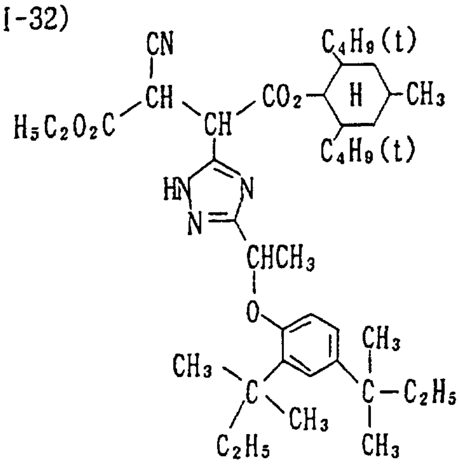

(II-33)

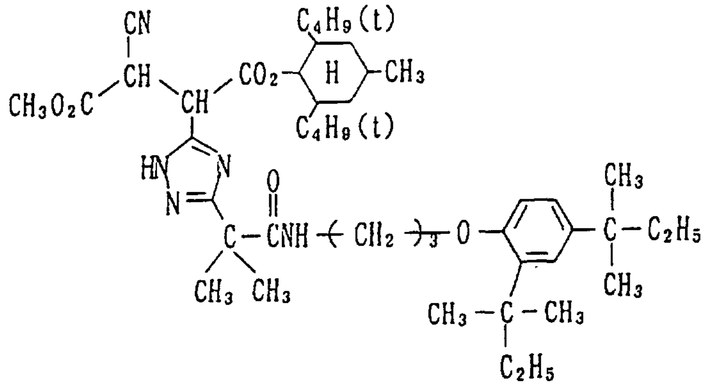

(II-34)

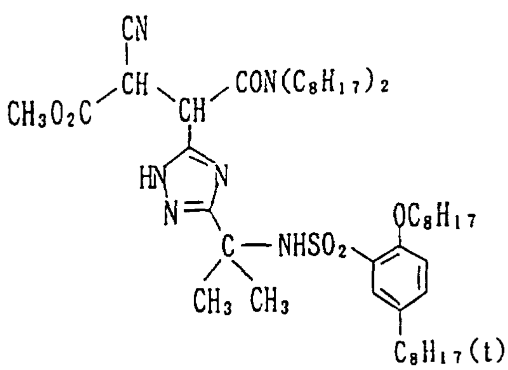

(II-35)

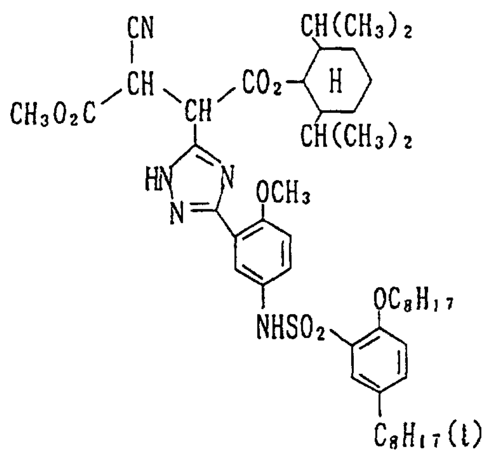

(II-36)

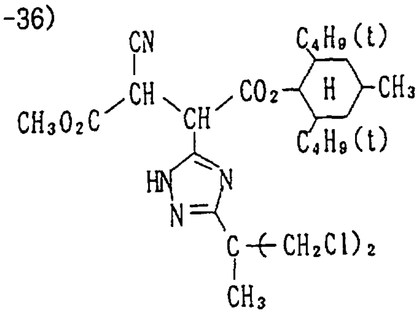

(II-37)

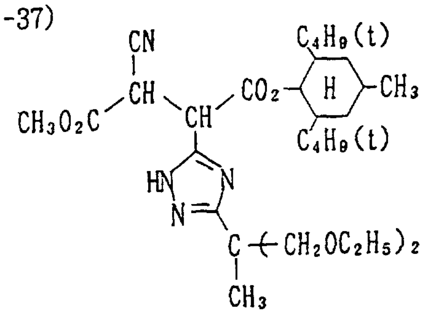

(II-38)

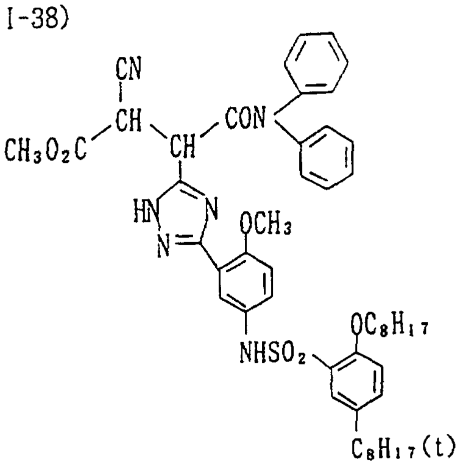

(II-39)

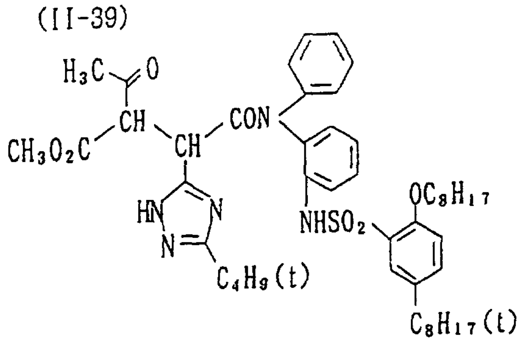

(II-40)

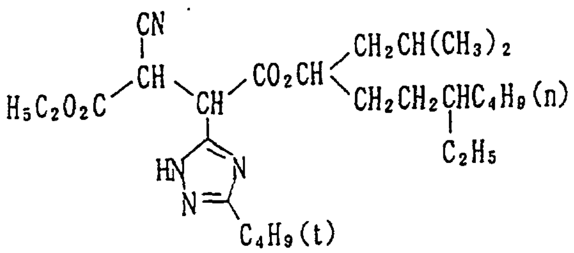

(II-41)

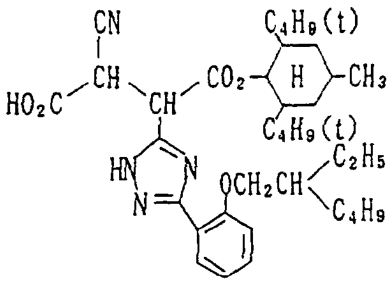

(II-42)

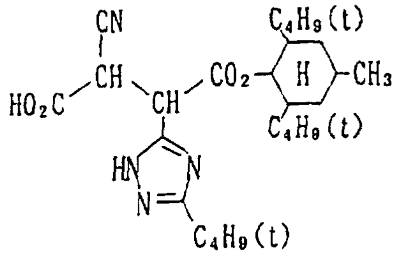

(II-43)

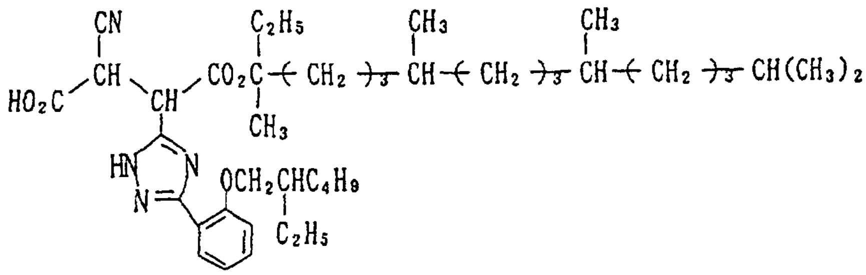

(II-44)

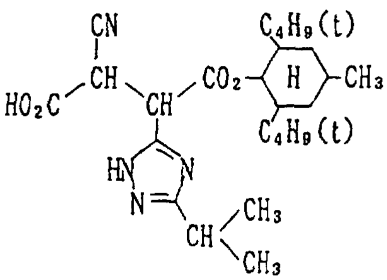

(II-45)

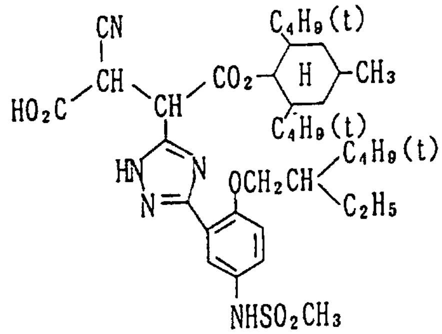

(II-46)

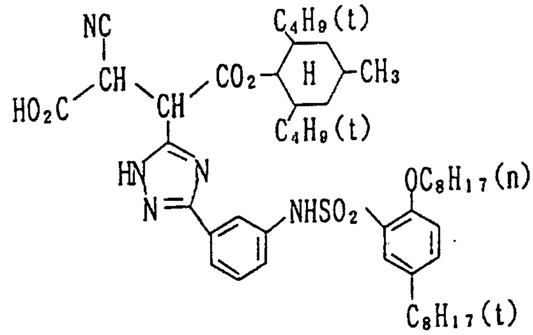

(II-47~49)

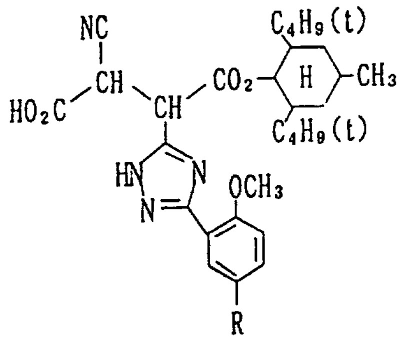

II−47 : R = $NHSO_2CH_3$

II−48 : R = $NHSO_2C_{12}H_{25}(n)$

II−49 : R = H

(III-2)

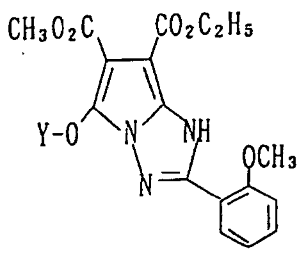

(III-1d) Y=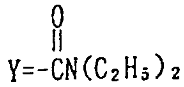

(III-3)

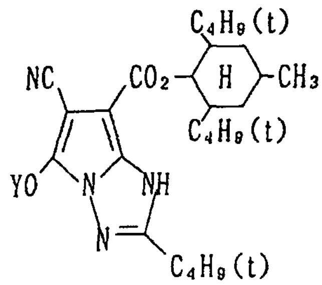

(III-3a) Y= 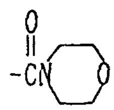

(III-3b) Y= 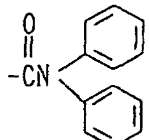

(III-3c) Y= 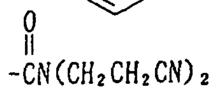

(III-3h) Y= 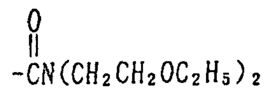

(III-3i) Y= 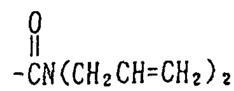

(III-3j) Y= 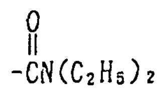

(III-3k) Y= 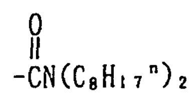

(III-3n) Y= 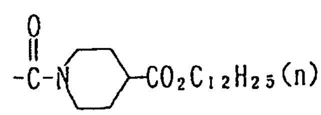

(III-5)

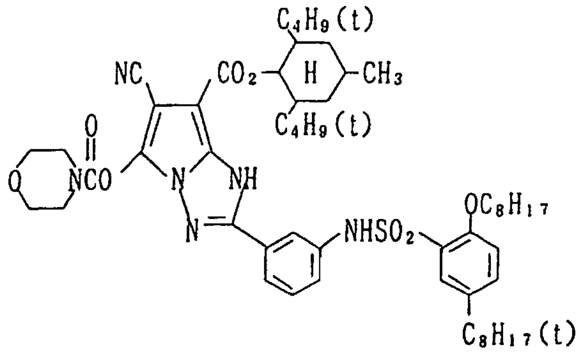

(III-8)

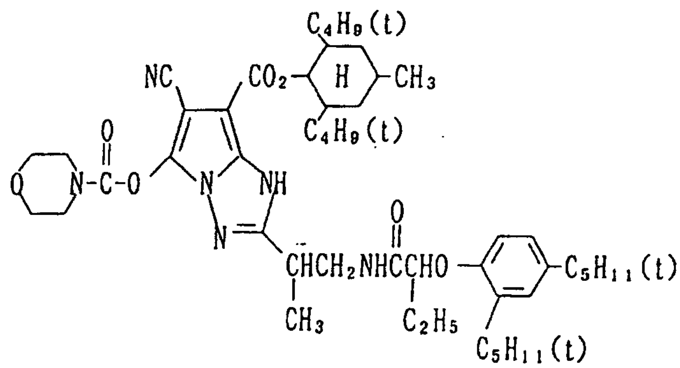

(III-12)

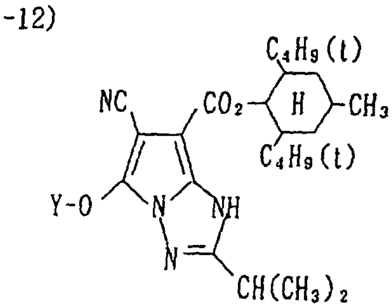

(III-12a) Y=

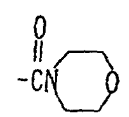

(III-12b) Y=

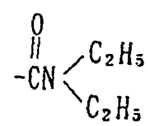

(III-16)

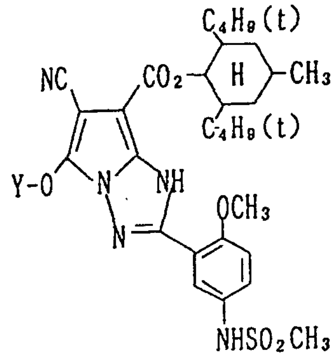

(III-16d) Y= 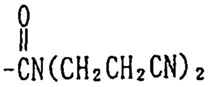

(III-16c) Y= 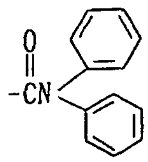

(III-18)

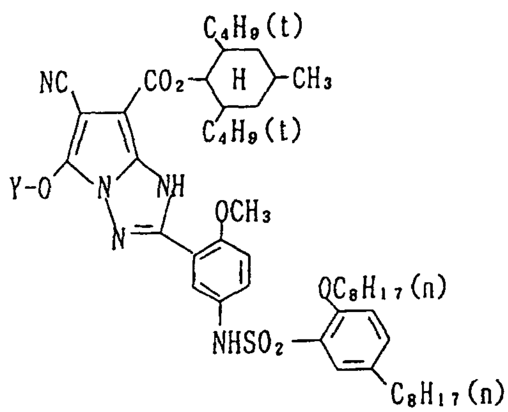

(III-18b) Y= 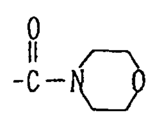

(III-18c) Y= 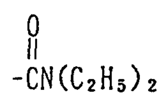

(III-21)

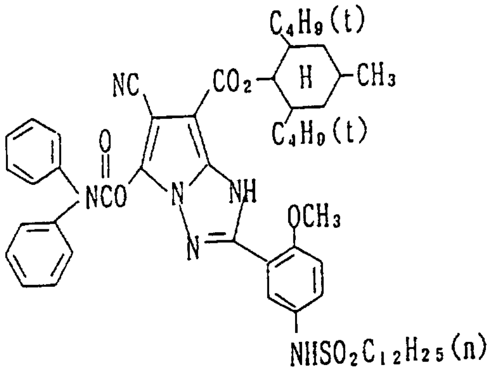

(III-22)

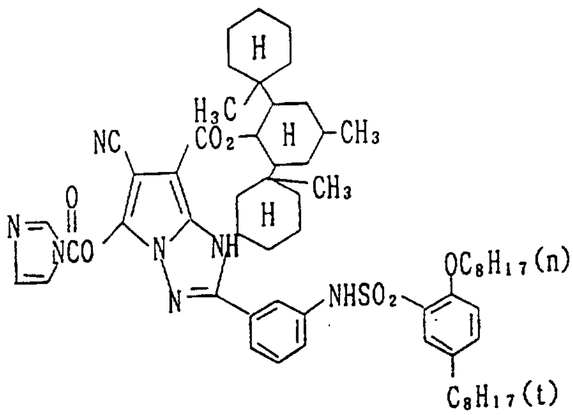

(III-23)

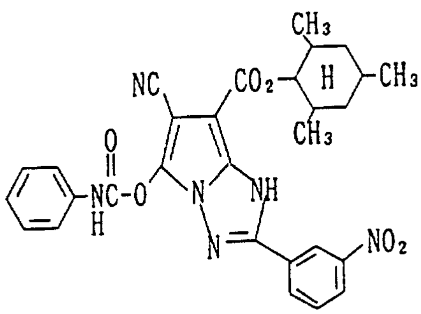

(III-24)

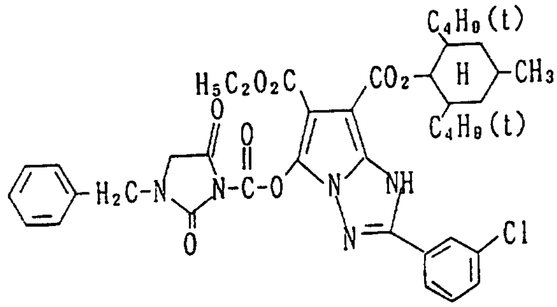

(III-25)

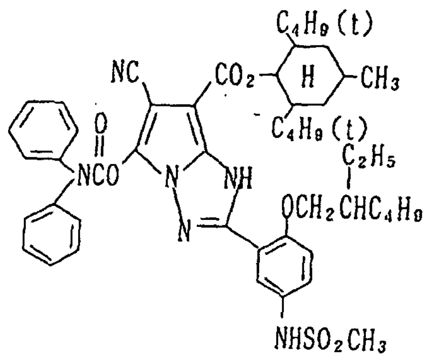

(III-26)

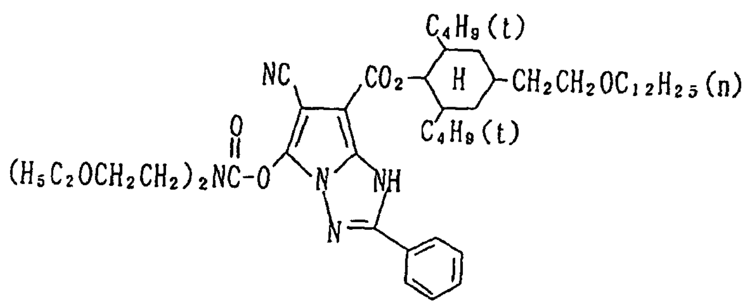

(III-29)

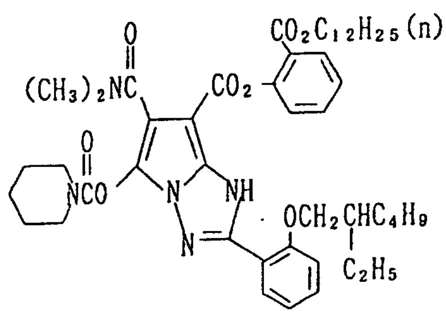

(III-34)

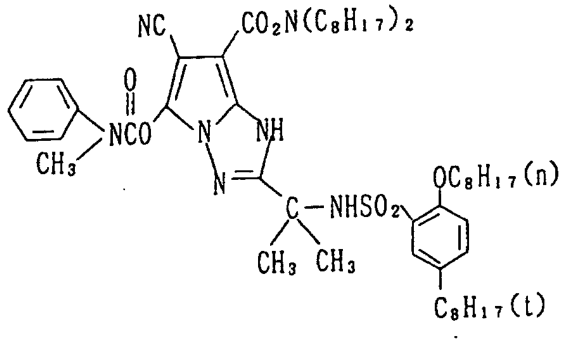

(III-35)

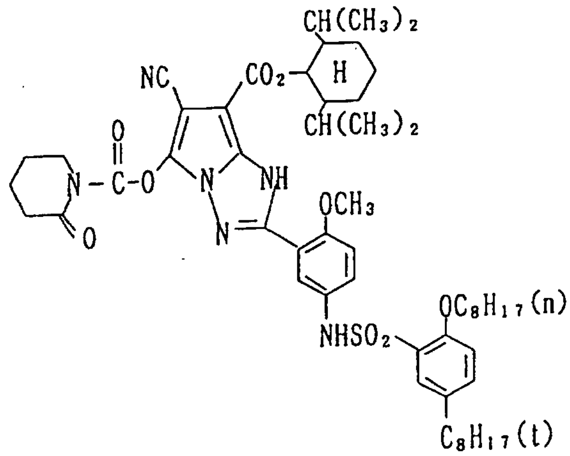

(III-36)

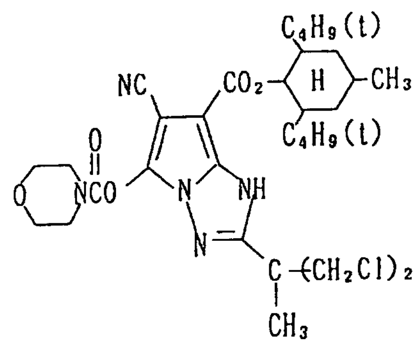

(III-37)

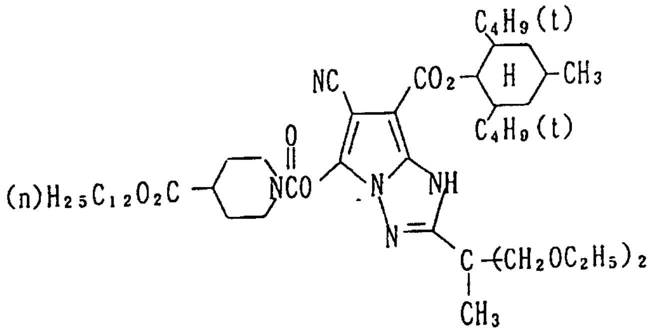

(III-40)

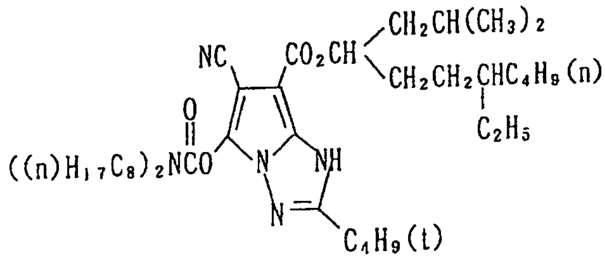

(III-43)

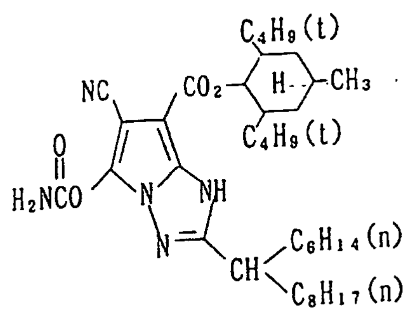

General processes for synthesizing compounds represented by formulae (I), (II), and (III) will be described below.

As illustrated in the scheme given below, a compound represented by general formula (I) can be obtained by halogenating a compound represented by formula (IV). (Hereinafter, this step is referred to as Step 1.)

A compound represented by formula (II) ($R_4$≠H) can be obtained by reacting the compound represented by formula (I) with an active methylene compound represented by $R_3$-$CH_2$-$CO_2R_4$ ($R_4$≠H) with the aid of an appropriate base. (Hereinafter, this step is referred to as Step 2.) A compound represented by formula (II) wherein $R_4$ is a hydrogen atom can be easily obtained from the thus-obtained corresponding ester through hydrolysis. (Hereinafter, this step is referred to as Step 3.)

By reacting the thus-obtained compound represented by formula (II) ($R_4$=H) with an acid halide (Y-X', wherein Y has the same meaning as defined hereinabove and X' represents a halogen atom) in the presence of a base, a 1H-pyrrolo[1,2-b][1,2,4]triazole represented by formula (III) can be obtained. (Hereinafter, this step is referred to as Step 4.)

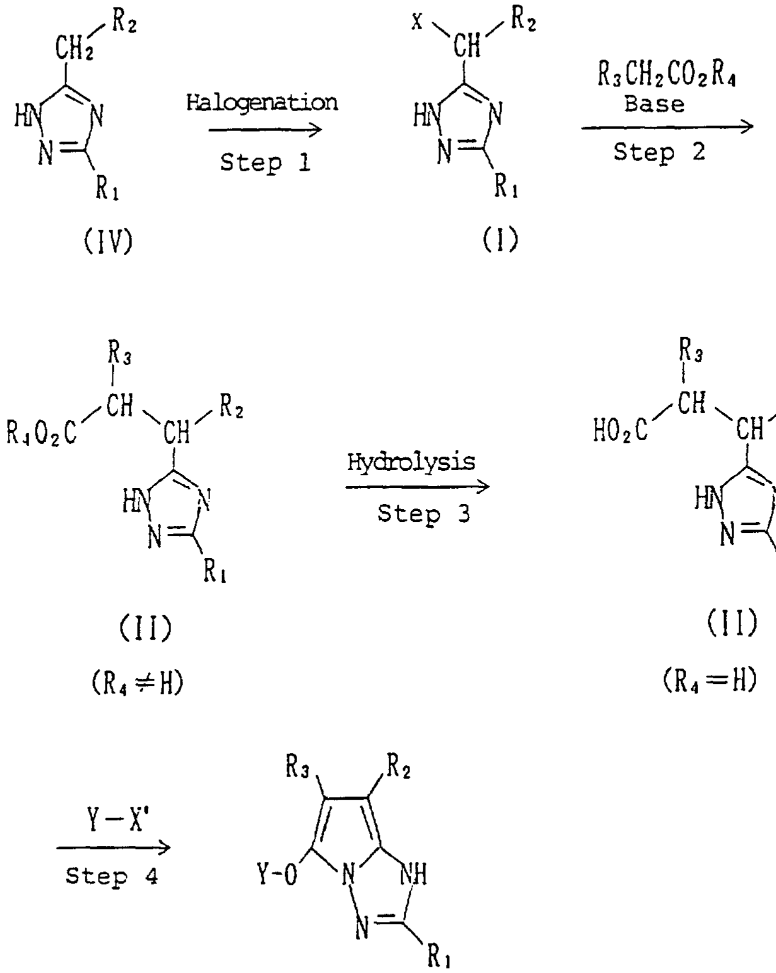


(IV) (I)

(II) ($R_4 \neq H$) (II) ($R_4 = H$)

Step 1 is described first in detail.

The triazole compound represented by formula (IV) which is used in Step 1 can be synthesized by any of known methods, e.g., the methods described in *J. C. S.*, 1961, p. 518, *J. C. S.*, 1962, p. 5149, *Angew. Chem.*, Vol. 72, p. 956 (1960), *Berichte.*, Vol. 97, p. 3436 (1964), the references cited therein or a method similar to any of such known methods.

Examples of the halogenating agent used for the halogenation in Step 1 include chlorine, sulfuryl chloride, copper(II) chloride, N-chlorosuccinimide, N-bromocaprolactam, N-bromophthalimide, 1,3-dibromo-5,5-dimethylhydantoin, bromine, N-bromosuccinimide, phenyltrimethylammonium tribromide, pyridinium bromide perbromide, and pyrrolidone hydrotribromide. Preferred of these are sulfuryl chloride, N-chlorosuccinimide, bromine, N-bromosuccinimide, and pyridinium bromide perbromide.

The molar ratio of the halogenating agent used in Step 1 to the triazole compound represented by formula (IV) is from 0.01 to 100, desirably from 0.5 to 10, preferably from 1.0 to 3.0.

Examples of solvents that can be used in Step 1 include methylene chloride, chloroform, 1,2-dichloroethane, acetonitrile, N,N-dimethylformamide, N,N-dimethylacetamide, dimethyl sulfoxide, N-methylpyrrolidone, dioxane, ethylacetate, tetrahydrofuran, water, carbon tetrachloride, acetic acid, methanol, ethanol, benzene, and pyridine.

The reaction temperature for Step 1 is from -78°C to 150°C, desirably from -20°C to 80°C, preferably from -10°C to 30°C.

The reaction time for Step 1 is from 1 minute to 72 hours, desirably from 15 minutes to 48 hours, preferably from 30 minutes to 36 hours.

Step 2 is explained below in detail.

Examples of the base for use in the nucleophilic substitution reaction of the compound represented by general formula (I) with the active methylene compound represented by $R_3CH_2CO_2R_4$ ($R_4 \neq H$) include n-butyllithium, t-butyllithium, lithium diisopropylamide, sodium hydride, potassium hydride, lithium hydride, potassium t-butoxide, sodium methoxide, potassium methoxide, lithium methoxide, sodium ethoxide, sodium amide, tetramethylguanidine, 1,8-diazabicyclo[5,4,0]-7-undecene (DBU) diisopropylethylamine, triethylamine, sodium hydroxide, and potassium hydroxide. Preferred of these are lithium diisopropylamide, n-butyllithium, sodium hydride, potassium t-butoxide, sodium t-butoxide, sodium methoxide, and sodium ethoxide.

The molar ratio of the base used in Step 2 to the active methylene compound is from 0.1 to 10, desirably from 0.5 to 2.0, preferably from 0.9 to 1.1.

The molar ratio of the active methylene compound used in Step 2 to the compound represented by formula (I) is from 0.01 to 100, desirably from 0.1 to 10, preferably from 0.5 to 2.0.

Examples of solvents that can be used in Step 2 include methylene chloride, chloroform, carbon tetrachloride, 1,2-dichloroethane, tetrahydrofuran, dioxane, benzene, toluene, xylene, petroleum ether, diethyl ether, N,N-dimethylformamide, N,N-dimethylacetamide and n-hexane. Preferred of these are methylene chloride, tetrahydrofuran, dioxane, toluene, and diethyl ether.

The reaction temperature for Step 2 is from -78°C to 150°C, desirably from -40°C to 50°C, preferably from -20°C to 30°C.

The reaction time for Step 2 is from 1 minute to 72 hours, desirably from 15 minutes to 48 hours, preferably from 30 minutes to 36 hours.

Step 3 is explained below in detail.

The hydrolysis of the ester moiety ($-CO_2R_4$) of the compound represented by formula (II) (Step 3) can be easily accomplished by an ordinary method (e.g., the method described in W. Green, *Protective Groups in Organic Synthesis*, John Wiley and Sons, Inc., New York 1981, pp. 152-178).

Step 4 is explained below in detail.

Examples of the base for use in Step 4 include triethylamine, diisopropylethylamine, pyridine, picoline, lutidine, collidine, 1,8-diazabicyclo[5,4,0]-7-undecene, 1,5-diazabicyclo[4,3,0]-5-nonene, sodium hydroxide, potassium hydroxide, sodium carbonate, and potassium carbonate. Preferred of these are triethylamine, diisopropylethylamine, pyridine, lutidine, picoline, and collidine.

The molar ratio of the base used in Step 4 to the compound represented by formula (II) ($R_4$=H) is from 0.01 to 1,000, desirably from 0.1 to 100, preferably from 2 to 50. However, this does not apply in the case of using these bases as a solvent.

Examples of the acid halide (Y-X', wherein Y has the same meaning as defined hereinabove and X' represents a halogen atom, preferably a chlorine atom) for use in Step 4 are as follows: dimethyl carbamyl chloride, diethylcarbamyl chloride, and di-n-butylcarbamyl chloride.

The molar ratio of the acid halide (Y-X') used in Step 4 to the compound represented by formula (II) ($R_4$=H) is from 0.01 to 100, desirably from 0.1 to 10, preferably from 2 to 5.

Examples of solvents that can be used in Step 4 include methylene chloride, chloroform, 1,2-dichloroethane, acetonitrile, ethyl acetate, N,N-dimethylformamide, N,N-dimethylacetamide, dimethyl sulfoxide, N-methylpyrrolidone, dioxane, tetrahydrofuran, carbon tetrachloride, benzene, and pyridine.

The reaction temperature for step 4 is from -78°C to 150°C, desirably from -40°C to 50°C, preferably from -10°C to 30°C.

The reaction time for step 4 is from 1 minute to 72 hours, desirably from 15 minutes to 48 hours, preferably from 30 minutes to 36 hours.

The present invention will be explained below in more detail by reference to Synthesis Examples.

## SYNTHESIS EXAMPLE 1

Synthesis of Compounds (I-1), (II-1), and (III-1)

According to the following schemes, Compounds (I-1), (II-1), and (III-1) were synthesized.

Scheme V

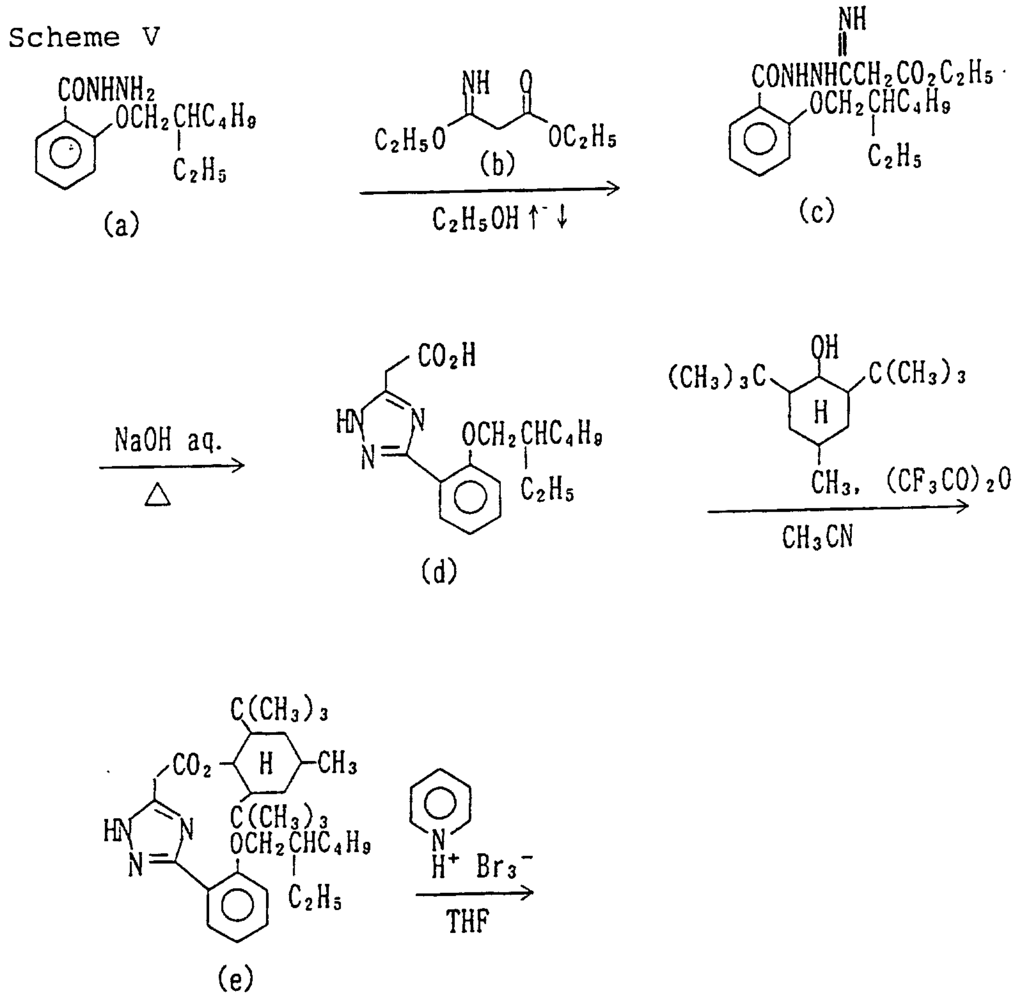

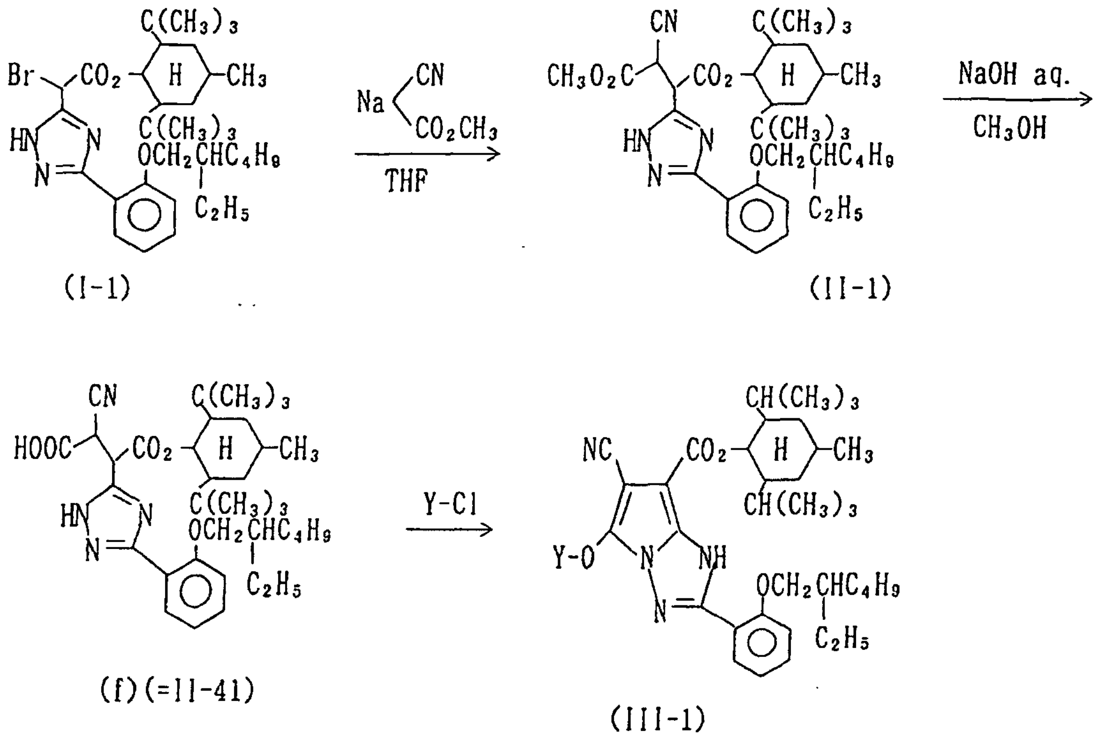


(I-1)

(II-1)

(f)(=II-41)

(III-1)

| | -Y |
|---|---|
| (III-1d) | 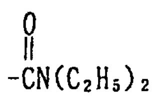 |

Synthesis of Compound (d)

A mixture of o-2-ethylhexyloxybenzhydrazide (a) (40 g, 151 mmol) and ethyl cyanoacetate imidate (b) (25 g, 155 mmol) was stirred in 100 ml of ethanol with refluxing for 5 hours. The ethanol was distilled off under a reduced pressure. To the residue was added 350 ml of an aqueous solution of sodium hydroxide (12.2 g, 306 mmol). After this

mixture was stirred with refluxing for 2 hours, 31 ml of concentrated hydrochloric acid was added to the reaction mixture. This reaction mixture was extracted with 200 ml of ethyl acetate. The organic phase was washed with water and dried with sodium sulfate. The solvent was then distilled off under a reduced pressure. The residue was recrystallized from an ethyl acetate/hexane mixture (1:1) to obtain Compound (d) (20 g, 60.4 mmol; 40%).

Synthesis of Compound (e)

To a solution in 200-ml acetonitrile of 2,6-di-t-butyl-4-methylcyclohexanol (17 g, 75 mmol) was added dropwise trifluoroacetic anhydride (10.6 ml, 75 mmol) at 0°C. Subsequently, Compound (d) (20 g, 60.4 mmol) was gradually added thereto. After the reaction mixture was stirred at room temperature for 2 hours, 300 ml of water was added. This reaction mixture was extracted with 300 ml of ethyl acetate. The organic phase was washed with aqueous sodium bicarbonate solution, water, and aqueous common salt solution. The washed organic phase was dried with sodium sulfate, and then concentrated under a reduced pressure. The residue was purified by column chromatography to obtain Compound (e) (18.5 g, 34.3 mmol; 57%).

Synthesis of Compound (I-1)

Pyridinium bromide perbromide (12.0 g, 37.7 mmol) was added to a tetrahydrofuran solution of Compound (e) (18.5 g, 34.3 mmol) at room temperature. This mixture was stirred for 8 hours. A solution in water (200 ml) of sodium sulfite (2 g) was added to the reaction mixture, which was then extracted with 300 ml of ethyl acetate. The organic phase was washed with water and aqueous common salt solution, dried with sodium sulfate, and then concentrated under a reduced pressure. The resulting residue was purified by column chromatography to obtain Compound (I-1) (16.0 g, 25.9 mmol; 75%; melting point, 132-133°C).

Synthesis of Compound (II-1)

To a solution in 50-ml tetrahydrofuran of methyl cyanoacetate (7.2 g, 73 mmol) was gradually added 60% sodium hydride (12.4 g, 60 mmol) at 0°C. This mixture was stirred at room temperature for 30 minutes (Solution $S_1$). Solution $S_1$ was added dropwise to a solution in tetrahydrofuran (75 ml) of Compound (I-1) (15.0 g, 24.3 mmol) at 0°C. This mixture was stirred at room temperature for 1 hour. Thereafter, 200 ml of 1 N hydrochloric acid and 200 ml of ethyl acetate were added to the reaction mixture to conduct extraction. The organic phase was washed with water and aqueous common salt solution, dried with sodium sulfate, and then concentrated under a reduced pressure. The resulting residue was purified by column chromatography to obtain Compound (II-1) as a light-yellow amorphous solid (12.7 g, 19.9 mmol; 82%). From its NMR spectrum, this Compound (II-1) was found to be a mixture of two isomers.

Synthesis of Compound (f)

Aqueous sodium hydroxide solution (5 g of NaOH and 50 ml of water) was added to a solution in 100-ml methanol of Compound (II-1) (10.5 g, 16.5 mmol). This mixture was stirred at 50°C for 2 hours. Thereafter, 200 ml of 1 N hydrochloric acid and 200 ml of ethyl acetate were added to the reaction mixture to conduct extraction. The organic phase was washed with water and aqueous common salt solution, and dried with sodium sulfate. The solvent was then distilled off under a reduced pressure. Subsequently, 100 ml of hexane was added to the resulting oil, upon which the oil crystallized. The crystals were recovered by filtration, and dried to obtain Compound (f) (=II-41) (9.5 g; 92%; melting point, 175-177°C).

Synthesis of Compound (III-1d)

Diethylcarbamyl chloride (1.0 ml, 1.8 mmol) was added dropwise to a solution in 20-ml pyridine of Compound (f) (2.0 g, 3.2 mmol) at 0°C. After this mixture was stirred at room temperature for 20 hours, 100 ml of ethyl acetate and 100 ml of 1 N hydrochloric acid were added. The organic phase was washed with 1 N hydrochloric acid, water, and aqueous common salt solution, and dried with sodium sulfate. The solvent was then distilled off under a reduced pressure. The residue was recrystallized from acetonitrile to obtain Compound (III-1d) (1.2 g, 1.7 mmol; 53%).

<u>SYNTHESIS EXAMPLE 2</u>

Synthesis of Compounds (I-3), (II-3) and (III-3)

Compound (g) was synthesized in the same manner as in Synthesis Example 1, and Compounds (I-3), (II-3), and (III-3) were synthesized therefrom according to the following scheme.

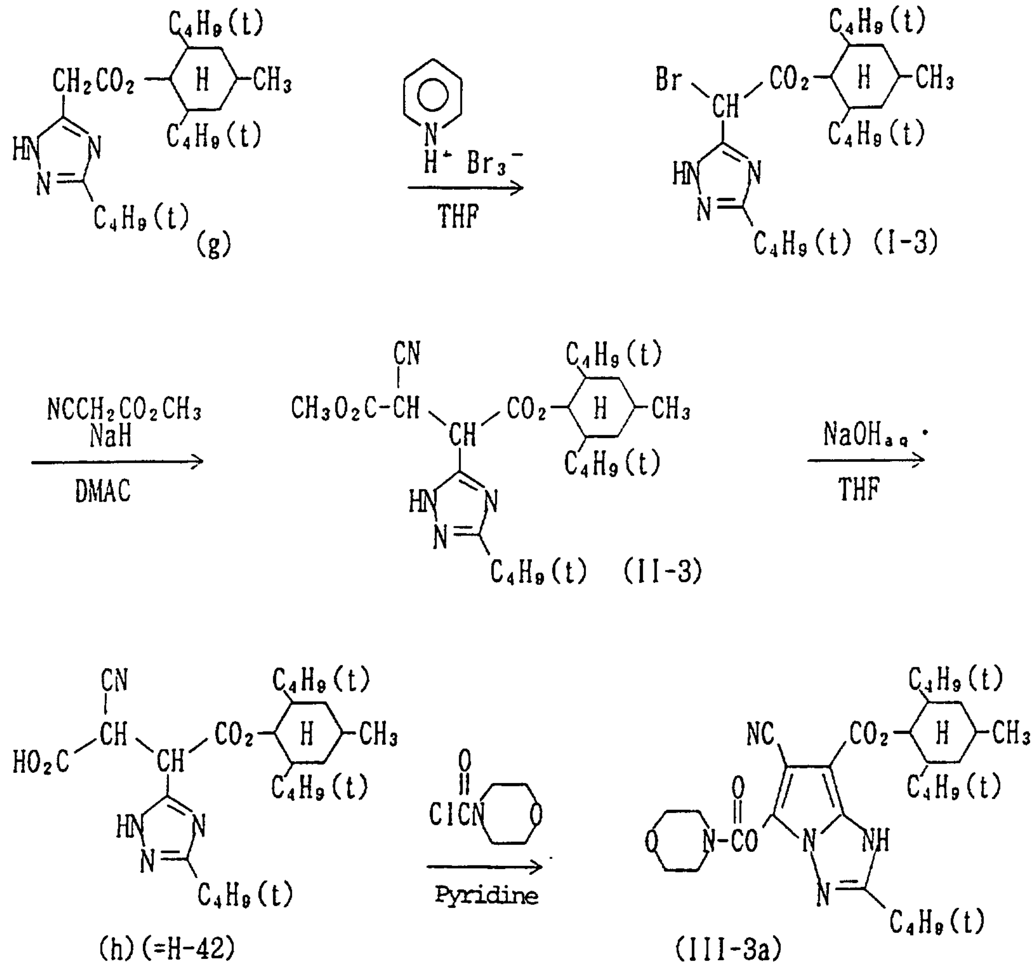


(g) (I-3) (II-3) (h)(=H-42) (III-3a)

Synthesis of Compound (I-3)

Pyridinium bromide perbromide (30 g, 93 mmol) was added to a solution in tetrahydrofuran (300 ml) of Compound (g) (28 g, 71.5 mmol) at room temperature. This mixture was stirred at room temperature for 10 hours. A solution in water (300 ml) of sodium sulfite (10 g) was added to the reaction mixture, and this mixture was stirred for further 2 hours. Ethyl acetate (500 ml) was added to the organic phase, which was then washed four times with a dilute aqueous solution of common salt. The washed organic phase was dried with sodium sulfate, and then concentrated under a reduced pressure. The residue was recrystallized from an ethyl acetate/hexane mixture to obtain Compound (I-3) (26.5 g, 56.3 mmol; 79%; melting point, 170-171°C).

Synthesis of Compound (II-3)

To a solution in dimethylformamide (20 ml) of methyl cyanoacetate (2.5 g, 25 mmol) was gradually added 60% sodium hydride (0.83 g, 21 mmol) at 0°C. This mixture was stirred at room temperature for 15 minutes (Solution $S_2$). Solution $S_2$ was added dropwise to a solution in dimethylformamide (35 ml) of Compound (I-3) (4.7 g, 10 mmol) at 0°C. This mixture was stirred at room temperature for 1 hour. Thereafter, 100 ml of 1 N hydrochloric acid and 100 ml of ethyl acetate were added to the reaction mixture to conduct extraction. The organic phase was washed with water and aqueous common salt solution, dried with sodium sulfate, and then concentrated under a reduced pressure. The residue was purified by column chromatography to obtain Compound (II-3) as a light-yellow amorphous solid (3.9 g,

7.9 mmol; 79%). From its NMR spectrum, this Compound (II-3) was found to be a mixture of two isomers.

Synthesis of Compound (h)

Aqueous sodium hydroxide solution (4 g of NaOH and 30 ml of water) was added to a solution in tetrahydrofuran (30 ml) of Compound (II-3) (3.9 g, 7.9 mmol). This mixture was stirred at room temperature for 3 hours, and then poured into 100 ml of dilute hydrochloric acid. The resulting mixture was extracted with ethyl acetate, and the resulting organic phase was washed with water (three times) and dried with sodium sulfate. Thereafter, the solvent was distilled off under a reduced pressure to obtain 3.7 g of crude compound (h). This crude compound (h) was used as it was in the subsequent step without conducting further purification.

Synthesis of Compound (III-3a)

Morpholinocarbamoyl chloride (2.16 g, 14.4 mmol) was added dropwise to a solution in pyridine (20 ml) of 3.7 g of crude compound (h) at 0°C. After this mixture was stirred at room temperature for 2 hours, 100 ml of ethyl acetate and 100 ml of 1 N hydrochloric acid were added. The organic phase was washed with 1 N hydrochloric acid, water, and aqueous common salt solution, and dried with sodium sulfate. The solvent was then distilled off under a reduced pressure. The residue was recrystallized from an ethyl acetate/hexane mixture to obtain Compound (III-3a) [3.3 g, 5.8 mmol; 80% (based on the acid chloride)]. With respect to Compounds (III-3b) to (III-3n), the desired compounds were able to be obtained in a high yield by the same method.

<u>SYNTHESIS EXAMPLE 3</u>

Synthesis of Compounds (I-6), (II-6), and (III-6)

Compound (i) was synthesized in the same manner as in Synthesis Example 1, and Compounds (I-6), (II-6), and (III-6) were synthesized therefrom according to the following scheme.

Compound (III-6) is not in accordance with the present invention.

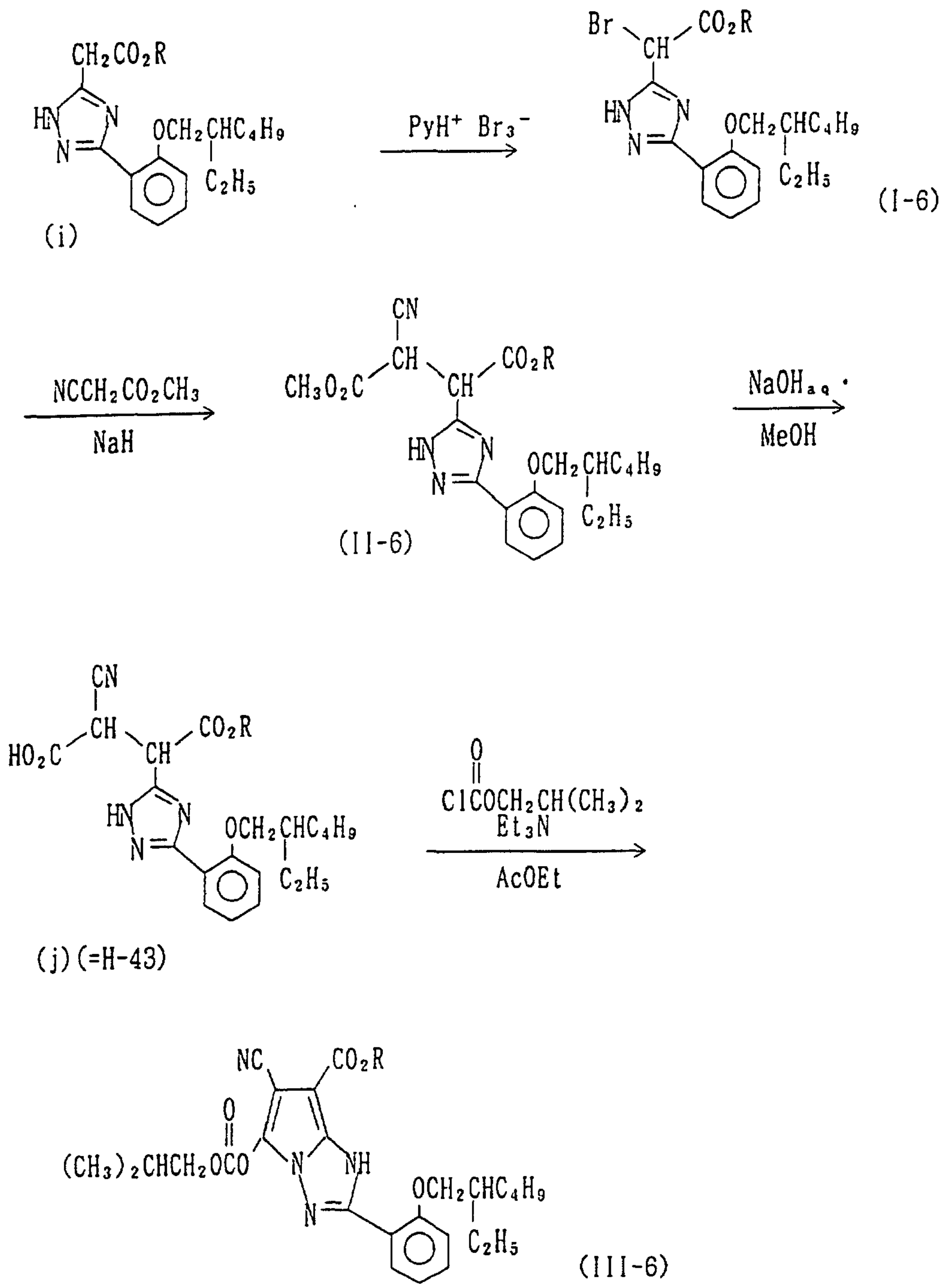


(i) (I-6) (II-6) (j)(=H-43) (III-6)

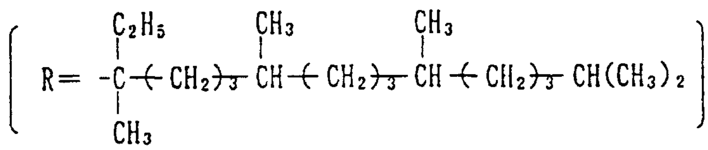

Synthesis of Compound (I-6)

Pyridinium bromide perbromide (2.5 g, 7.9 mmol) was added to a solution in tetrahydrofuran (50 ml) of Compound (i) (3.7 g, 6.05 mmol) at room temperature. This mixture was stirred at room temperature for 10 hours. To the reaction mixture was added 10 ml of a saturated aqueous solution of sodium sulfite. This mixture was stirred at room temperature for further 30 minutes. Ethyl acetate (100 ml) was added to the organic phase, which was then washed four times with a dilute aqueous solution of common salt. The washed organic phase was dried with sodium sulfate, and then concentrated under a reduced pressure. The residue was purified by column chromatography to obtain Compound (I-6) as a colorless oily substance (2.9 g, 4.20 mmol; 69%).

Synthesis of Compound (II-6)

To a solution in tetrahydrofuran (10 ml) of methyl cyanoacetate (1.2 g, 12 mmol) was gradually added 60% sodium hydride (0.4 g, 10 mmol) at 0°C. This mixture was stirred at room temperature for 15 minutes (Solution $S_3$). Solution $S_3$ was added dropwise to a solution in tetrahydrofuran (30 ml) of Compound (I-6) (2.8 g, 4 mmol) at 0°C. This mixture was stirred at room temperature for 30 minutes. Thereafter, 100 ml of 1 N hydrochloric acid and 100 ml of ethyl acetate were added to the reaction mixture to conduct extraction. The organic phase was washed with water and aqueous common salt solution, dried with sodium sulfate, and then concentrated under a reduced pressure. The residue was purified by column chromatography to obtain Compound (II-6) (2.35 g, 3.3 mmol; 83%). From its NMR spectrum, this Compound (II-6) was found to be a mixture of two isomers.

Synthesis of Compound (III-6)

Aqueous sodium hydroxide solution (1 g of NaOH and 10 ml of water) was added to a solution in methanol (20 ml) of Compound (II-6) (2.0 g, 28.3 mmol). This mixture was stirred at room temperature for 2 hours, and then poured into 100 ml of 1 N hydrochloric acid. The resulting mixture was extracted with 100 ml of ethyl acetate. The organic phase was washed with water (three times), and dried with sodium sulfate. Isobutyl chloroformate (9.3 ml, 7.1 mmol) was added dropwise to the dried solution at 0°C. Subsequently, triethylamine (1.0 ml, 7.1 mmol) was added thereto dropwise. The reaction mixture was stirred at 0°C for 1 hour, and then poured into 100 ml of 1 N hydrochloric acid. The organic phase was washed with water (three times), and then purified by column chromatography to obtain Compound (III-6) (1.1 g, 1.42 mmol; 50% in two steps).

The melting points and the $pK_a$ values measured in a 6/4 mixture (by volume) of tetrahydrofuran and water at 25°C of representative examples of the compound represented by general formula (III) according to the present invention are shown in Table A along with those properties of comparative examples. For the purpose of comparison in thermal stability, the compound amount (%) remaining after 1-day standing at 100°C (in a sealed chamber) is also shown with respect to part of those examples.

Table A

| Compound No. | Melting point (°C) | $pK_a$ | Thermal stability (%) | Remarks |
|---|---|---|---|---|
| III-1x | - | 9.48 | 98 | Comparative |
| III-1y | 175-176 | 8.09 | 90 | " |
| III-1d | 196-197 | 9.36 | 98 | Invention |
| III-3x | - | 8.71 | 97 | Comparative |
| III-3y | 239-240 | 7.52 | 69 | " |
| III-3a | 268-272 | 8.28 | 95 | Invention |
| III-3b | 167 | 8.42 | 95 | " |
| III-3c | 230-231 | 7.97 | 94 | " |
| III-3h | 85 | 8.43 | - | " |
| III-3i | 209-210 | 8.61 | - | " |
| III-3j | 148-150 | 8.66 | - | " |
| III-3k | 170-171 | 8.90 | - | " |
| III-3n | 172-173 | 8.75 | - | " |
| III-5x | - | 7.26 | - | Comparative |
| III-5y | - | 5.90 | - | " |
| III-5 | 212-214 | 6.72 | - | Invention |
| III-6x | - | 9.38 | - | Comparative |
| III-6y | - | 7.97 | - | " |
| III-6 | 55-57 | 8.86 | - | " |

Table A (Continued)

| Compound No. | Melting point (°C) | $pK_a$ | Thermal stability (%) | Remarks |
|---|---|---|---|---|
| III-12x | - | 8.63 | 97 | Comparative |
| III-12y | - | 7.40 | 64 | " |
| III-12a | 254-247 | 8.20 | 95 | Invention |
| III-12b | 199-200 | 8.61 | 97 | " |
| III-16x | - | 8.26 | - | Comparative |
| III-16y | - | 6.94 | - | " |
| III-16c | 260-262 | 7.80 | - | Invention |
| III-16d | 217-218 | 7.30 | - | " |
| III-18x | - | 8.79 | - | Comparative |
| III-18y | 260-262 | 7.54 | - | " |
| III-18b | - | 8.15 | - | Invention |
| III-18c | - | 8.67 | - | " |
| III-21x | - | 8.35 | - | Comparative |
| III-21y | - | 6.97 | - | " |
| III-21 | 174-175 | 7.90 | - | Invention |
| III-25x | - | 8.47 | - | Comparative |
| III-25y | - | 7.01 | - | " |
| III-25 | 247-249 | 8.04 | - | Invention |

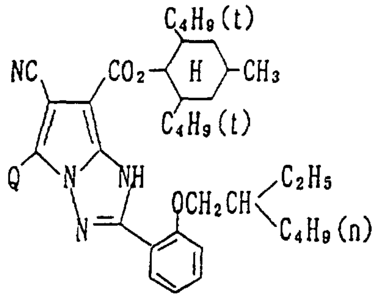

III-1x Q=H

III-1y Q=Cl

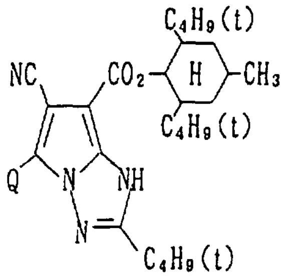

III-3x Q=H

III-3y Q=Cl

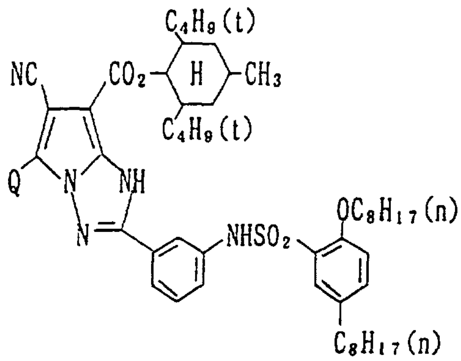

III-5x Q=H

III-5y Q=Cl

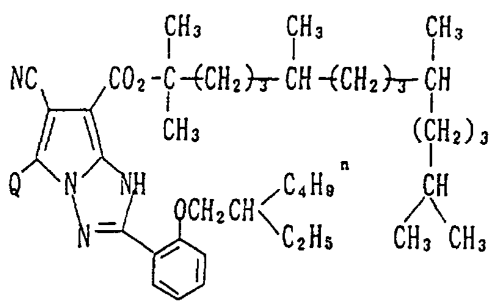

III-6x Q=H

III-6y Q=Cl

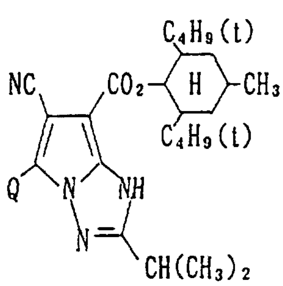

III-12x Q=H

III-12y Q=Cl

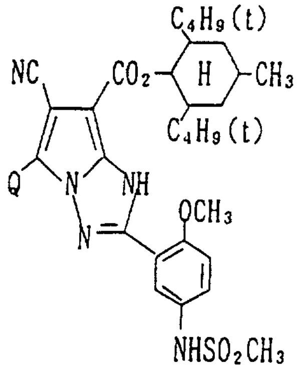

III-16x Q=H
III-16y Q=Cl

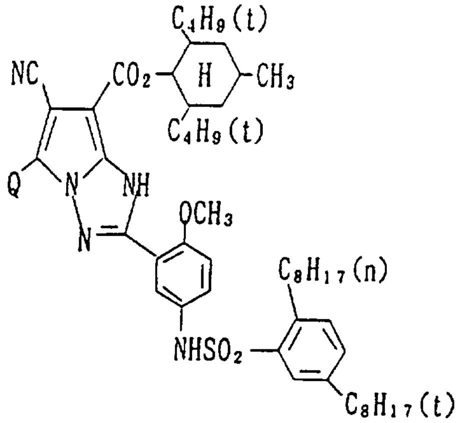

III-18x Q=H
III-18y Q=Cl

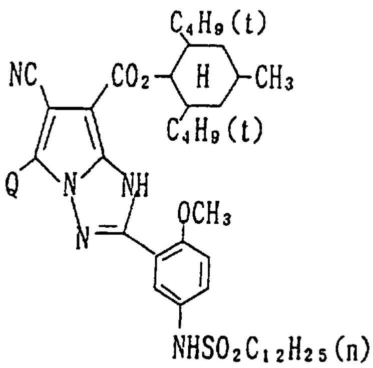

III-21x Q=H
III-21y Q=Cl

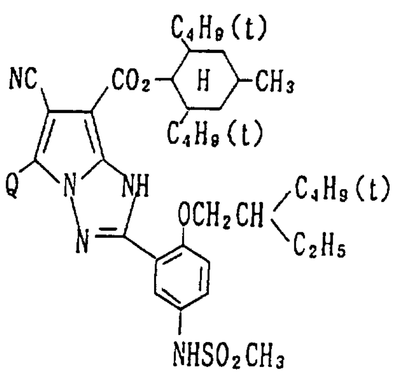

III-25x Q=H
III-25y Q=Cl

The novel 1H-pyrrolo[1,2-b][1,2,4]triazole derivative represented by formula (III), which is useful as an intermediate for physiologically active substances such as medicines and agricultural chemicals or as a precursor for photographic cyan couplers, dyes for dye-providing heat transfer materials, and filter dyes for use in solid camera tubes or liquid-crystal color displays, can be efficiently synthesized by using as intermediates the novel 1H-1,2,4-triazole derivatives of this invention which are respectively represented by formulae (I) and (II). The 1H-pyrrolo[1,2-b][1,2,4] triazole derivative represented by formula (III) according to the present invention has a $pK_a$ value higher by about 1 than that of the 5-chloro-substituted corresponding compound and is thermally stable.

## Claims

**1.** A 1H-1,2,4-triazole derivative represented by formula (I)

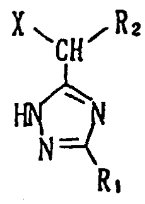

(I)

wherein $R_1$ represents an aliphatic group which may be substituted or an aryl group which may be substituted; $R_2$ represents an electron-withdrawing group having a Hammett's substituent constant $\sigma_p$ of from 0.2 to 1.0; and X represents a halogen atom.

**2.** The 1H-1,2,4-triazole derivative as claimed in claim 1, wherein the aliphatic group represented by $R_1$ represents a linear or branched alkyl group having 1 to 36 carbon atoms, an aralkyl group having up to 36 carbon atoms, an alkenyl group having up to 36 carbon atoms, an alkynyl group having up to 36 carbon atoms, a cycloalkyl group having up to 36 carbon atoms, or a cycloalkenyl group having up to 36 carbon atoms.

**3.** The 1H-1,2,4-triazole derivative as claimed in claim 1 or 2, wherein $R_2$ represents an acyl group, a acyloxy group, a carbamoyl group, an aliphatic oxycarbonyl group, an aryloxycarbonyl group, a cyano group, a nitro group, a dialkylphosphono group, a diarylphosphono group, a diarylphosphinyl group, an alkylsulfinyl group, an arylsulfinyl group, an alkylsulfonyl group, an arylsulfonyl group, a sulfonyloxy group, an acylthio group, a sulfamoyl group, a thiocyanate group, a thiocarbonyl group, an alkyl group substituted with at least two halogen atoms, an alkoxy group substituted with at least two halogen atoms, an aryloxy group substituted with at least two halogen atoms, an alkylamino group substituted with at least two halogen atoms, an alkylthio group substituted with at least two halogen atoms, an aryl group substituted with another electron-withdrawing group having a $\sigma_p$ value of 0.20 or higher, a heterocyclic group, a chlorine atom, a bromine atom, an azo group, or a selenocyanate group.

**4.** The 1H-1,2,4-triazole derivative as claimed in claim 1 or 2, wherein $R_2$ is an aliphatic oxycarbonyl group which may be substituted.

**5.** The 1H-1,2,4-triazole derivative as claimed in claim 1 or 2, wherein $R_2$ is an aliphatic oxycarbonyl group represented by formula (V)

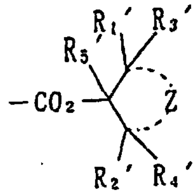

(V)

wherein $R_1$' and $R_2$' each represents an aliphatic group; $R_3$', $R_4$' and $R_5$' each represents a hydrogen atom or an aliphatic group; and Z represents a group of non-metallic atoms necessary for forming a 5- to 8-membered ring.

**6.** The 1H-1,2,4-triazole derivative as claimed in any one of claims 1 to 5, wherein X is a bromine atom or a chlorine atom.

**7.** The 1H-1,2,4-triazole derivative as claimed in claim 1, which is represented by formula (I')

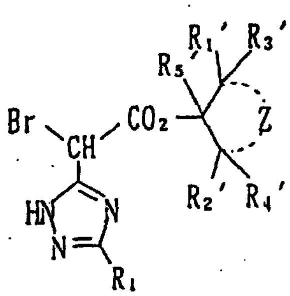

(I')

wherein $R_1$ represents an aliphatic group which may be substituted or an aryl group which may be substituted; $R_1'$ and $R_2'$ each represents an aliphatic group; $R_3'$, $R_4'$ and $R_5'$ each represents a hydrogen atom or an aliphatic group; and Z represents a group of non-metallic atoms necessary for forming a 5- to 8-membered ring.

**8.** A 1H-1,2,4-triazole derivative represented by formula (II)

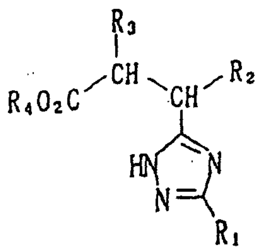

(II)

wherein $R_1$ represents an aliphatic group which may be substituted or an aryl group which may be substituted; $R_2$ and $R_3$ each represents an electron-withdrawing group having a Hammett's substituent constant $\sigma_p$ of from 0.2 to 1.0; and $R_4$ represents a hydrogen atom, an aliphatic group, or an aryl group.

**9.** The 1H-1,2,4-triazole derivative as claimed in claim 8, wherein the aliphatic group represented by $R_1$ represents a linear or branched alkyl group having 1 to 36 carbon atoms, an aralkyl group having up to 36 carbon atoms, an alkenyl group having up to 36 carbon atoms, an alkynyl group having up to 36 carbon atoms, a cycloalkyl group having up to 36 carbon atoms, or a cycloalkenyl group having up to 36 carbon atoms.

**10.** The 1H-1,2,4-triazole derivative as claimed in claim 8 or 9, wherein $R_2$ and $R_3$ each represents an acyl group, a acyloxy group, a carbamoyl group, an aliphatic oxycarbonyl group, an aryloxycarbonyl group, a cyano group, a nitro group, a dialkylphosphono group, a diarylphosphono group, a diarylphosphinyl group, an alkylsulfinyl group, an arylsulfinyl group, an alkylsulfonyl group, an arylsulfonyl group, a sulfonyloxy group, an acylthio group, a sulfamoyl group, a thicyanate group, a thiocarbonyl group, an alkyl group substituted with at least two halogen atoms, an alkoxy group substituted with at least two halogen atoms, an aryloxy group substituted with at least two halogen atoms, an alkylamino group substituted with at least two halogen atoms, an alkylthio group substituted with at least two halogen atoms, an aryl group substituted with another electron-withdrawing group having a $\sigma_p$ value of 0.20 or higher, a heterocyclic group, a chlorine atom, a bromine atom, an azo group, or a selenocyanate group.

**11.** The 1H-1,2,4-triazole derivative as claimed in claim 8 or 9, wherein $R_2$ is an aliphatic oxycarbonyl group which may be substituted and $R_3$ is a cyano group.

**12.** The 1H-1,2,4-triazole derivative as claimed in claim 8 or 9, wherein $R_2$ is an aliphatic oxycarbonyl group represented by formula (V)

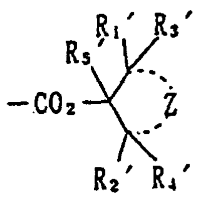

(V)

wherein $R_1$' and $R_2$' each represents an aliphatic group; $R_3$', $R_4$' and $R_5$' each represents a hydrogen atom or an aliphatic group; and Z represents a group of non-metallic atoms necessary for forming a 5- to 8-membered ring.

**13.** The 1H-1,2,4-triazole derivative as claimed in any one of claims 8 to 12, wherein $R_3$ represents a cyano group, an aliphatic oxycarbonyl group, a dialkylphosphono group, an alkylsulfonyl group, an arylsulfonyl group, or a fluoroalkyl group.

**14.** The 1H-1,2,4-triazole derivative as claimed in any one of claims 8 to 13, wherein $R_4$ is an hydrogen atom or an alkyl group.

**15.** The 1H-1,2,4-triazole derivative as claimed in claim 8, which is represented by formula (II')

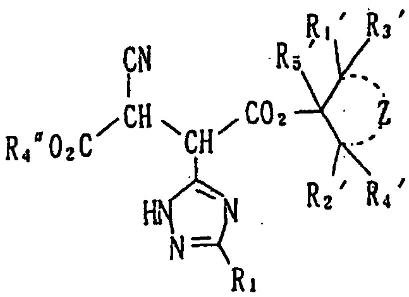

(II')

wherein $R_1$ represents an aliphatic group which may be substituted or an aryl group which may be substituted; $R_1$' and $R_2$' each represents an aliphatic group; $R_3$', $R_4$' and $R_5$' each represents a hydrogen atom or an aliphatic group; $R_4$'' represents a hydrogen atom, or an alkyl group having from 1 to 7 carbon atoms; and Z represents a group of non-metallic atoms necessary for forming a 5- to 8-membered ring.

**16.** A process for producing the 1H-1,2,4-triazole derivative as claimed in claim 8, which comprises:

(i) reacting a 1H-1,2,4-triazole derivative represented by formula (I) with an active methylene compound represented by $R_3$-$CH_2$-$CO_2R_4$''' in the presence of a base to obtain a 1H-1,2,4-triazole derivative represented by formula (II) wherein $R_4$ is not a hydrogen atom:

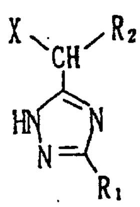

(I)

wherein $R_1$ represents an aliphatic group which may be substituted or an aryl group which may be substituted;

$R_2$ represents an electron-withdrawing group having a Hammett's substituent constant $\sigma_p$ of from 0.2 to 1.0; and X represents a halogen atom, and wherein $R_3$ represents a cyano group, an aliphatic oxycarbonyl group, a dialkylphosphono group, an alkylsulfonyl group, an arylsulfonyl group, or a fluoroalkyl group; and $R_4'''$ represents an aliphatic group or an aryl group; or
(ii) optionally hydrolyzing the 1H-1,2,4-triazole derivative represented by formula (II) wherein $R_4$ is not a hydrogen atom to obtain a 1H-1,2,4-triazole derivative represented by formula (II) wherein $R_4$ is a hydrogen atom.

**17.** A 1H-pyrrolo[1,2-b][1,2,4]triazole derivative represented by formula (III)

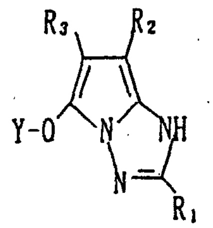

(III)

wherein $R_1$ represents an aliphatic group which may be substituted or an aryl group which may be substituted; $R_2$ and $R_3$ each represents an electron-withdrawing group having a Hammett's substituent constant $\sigma_p$ of from 0.2 to 1.0; Y represents -C(=O)-N(Ra')Ra and wherein
Ra and Ra' each represents a hydrogen atom, an aliphatic group which may be substituted, or an aryl group which may be substituted, and Ra and Ra' may be bonded to each other to form a ring.

**18.** The 1H-pyrrolo[1,2-b][1,2,4]triazole derivative as claimed in claim 17, wherein the aliphatic group represented by $R_1$ represents a linear or branched alkyl group having 1 to 36 carbon atoms, an aralkyl group having up to 36 carbon atoms, an alkenyl group having up to 36 carbon atoms, an alkynyl group having up to 36 carbon atoms, a cycloalkyl group having up to 36 carbon atoms, or a cycloalkenyl group having up to 36 carbon atoms.

**19.** The 1H-pyrrolo[1,2-b][1,2,4]triazole derivative as claimed in claim 17 or 18, wherein $R_2$ and $R_3$ each represents an acyl group, a acyloxy group, a carbamoyl group, an aliphatic oxycarbonyl group, an aryloxycarbonyl group, a cyano group, a nitro group, a dialkylphosphono group, a diarylphosphono group, a diarylphosphinyl group, an alkylsulfinyl group, an arylsulfinyl group, an alkylsulfonyl group, an arylsulfonyl group, a sulfonyloxy group, an acylthio group, a sulfamoyl group, a thiocyanate group, a thiocarbonyl group, an alkyl group substituted with at least two halogen atoms, an alkoxy group substituted with at least two halogen atoms, an aryloxy group substituted with at least two halogen atoms, an alkylamino group substituted with at least two halogen atoms, an alkylthio group substituted with at least two halogen atoms, an aryl group substituted with another electron-withdrawing group having a $\sigma_p$ value of 0.20 or higher, a heterocyclic group, a chlorine atom, a bromine atom, an azo group, or a selenocyanate group.

**20.** The 1H-pyrrolo[1,2-b][1,2,4]triazole derivative as claimed in claim 17 or 18, wherein $R_2$ is an aliphatic oxycarbonyl group which may be substituted and $R_3$ is a cyano group.

**21.** The 1H-pyrrolo[1,2-b][1,2,4]triazole derivative as claimed in claim 17 or 18, wherein $R_2$ is an aliphatic oxycarbonyl group represented by formula (V)

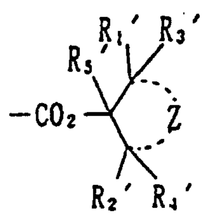

(V)

wherein $R_1$' and $R_2$' each represents an aliphatic group; $R_3$', $R_4$' and $R_5$' each represents a hydrogen atom or an aliphatic group; and Z represents a group of non-metallic atoms necessary for forming a 5- to 8-membered ring.

**22.** The 1H-pyrrolo[1,2-b][1,2,4]triazole derivative as claimed in any one of claims 17 to 21, wherein $R_3$ represents a cyano group, an aliphatic oxycarbonyl group, a dialkylphosphono group, an alkylsulfonyl group, an arylsulfonyl group, or a fluoroalkyl group.

**23.** The 1H-pyrrolo[1,2-b][1,2,4]triazole derivative as claimed in claim 17 or 18, which is represented by formula (III')

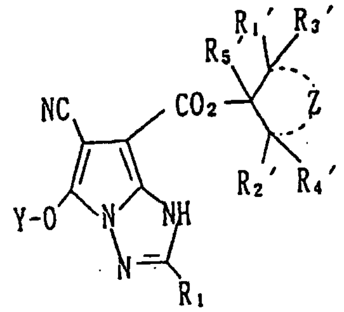

(III')

wherein $R_1$ represents an aliphatic group which may be substituted or an aryl group which may be substituted;
$R_1$' and $R_2$' each represents an aliphatic group;
$R_3$', $R_4$' and $R_5$' each represents a hydrogen atom or an aliphatic group;
Y represents -C(=O)-N(Ra')Ra and wherein
Ra and Ra' each represents a hydrogen atom, an aliphatic group which may be substituted, or an aryl group which may be substituted, and Ra and Ra' may be bonded to each other to form a ring; and
Z represents a group of non-metallic atoms necessary for forming a 5- to 8-membered ring.

**24.** A process for producing a 1H-pyrrolo[1,2-b][1,2,4]triazole derivative represented by formula (III-a)

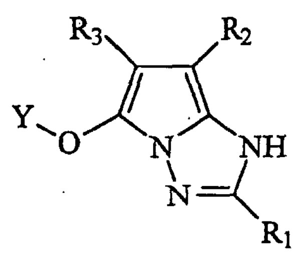

(III-a)

which comprises reacting a 1H-1,2,4-triazole derivative represented by formula (II") with an acid halide represented by Y-X' in the presence of a base:

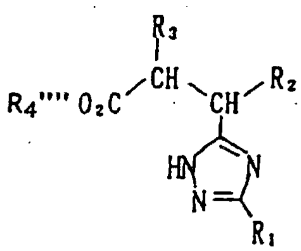

(II'')

wherein $R_1$ represents an aliphatic group which may be substituted or an aryl group which may be substituted; $R_2$ and $R_3$ each represents an electron-withdrawing group having a Hammett's substituent constant $\sigma_p$ of from 0.2 to

1.0; and $R_4''''$ represents a hydrogen atom; and
wherein Y represents -C(=O)-$R_5$, -$SO_2R_6$ or -P(=O)($R_7$)($R_8$), in which $R_5$, $R_7$ and $R_8$ each represents a hydrogen atom, an aliphatic group which may be substituted, an aryl group which may be substituted, an aliphatic oxy group which may be substituted, an aryloxy group which may be substituted, or a group represented by - N(Ra')Ra, and $R_7$ and $R_8$ may be bonded to each other to form a ring; $R_6$ represents an aliphatic group which may be substituted, an aryl group which may be substituted, or a group represented by -N(Ra')Ra; and wherein Ra and Ra' each represents a hydrogen atom, an aliphatic group which may be substituted, or an aryl group which may be substituted, and Ra and Ra' may be bonded to each other to form a ring; and X' represents a halogen atom.

**25.** A process for producing a 1H-pyrrolo[1,2-b][1,2,4]triazole derivative represented by formula (III-a)

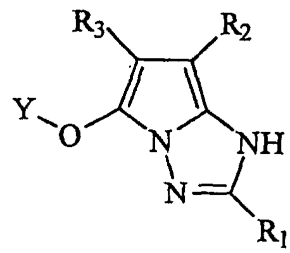

(III-a)

which comprises:

(i) reacting a 1H-1,2,4-triazole derivative represented by formula (I) with an active methylene compound represented by $R_3$-$CH_2$-$CO_2R_4'''$ in the presence of a base to obtain a 1H-1,2,4-triazole derivative represented by formula (II) wherein $R_4$ is not a hydrogen atom:

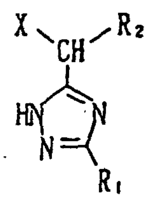

(I)

wherein $R_1$ represents an aliphatic group which may be substituted or an aryl group which may be substituted; $R_2$ represents an electron-withdrawing group having a Hammett's substituent constant $\sigma_p$ of from 0.2 to 1.0; and X represents a halogen atom, and wherein $R_3$ represents a cyano group, an aliphatic oxycarbonyl group, a dialkylphosphono group, an alkylsulfonyl group, an arylsulfonyl group, or a fluoroalkyl group; and $R_4'''$ represents an aliphatic group or an aryl group;

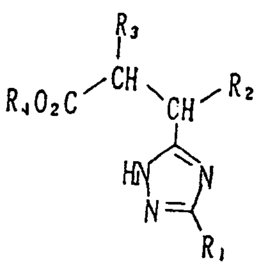

(II)

wherein $R_1$ represents an aliphatic group which may be substituted or an aryl group which may be substituted; $R_2$ and $R_3$ each represents an electron-withdrawing group having a Hammett's substituent constant $\sigma_p$ of from 0.2 to 1.0; and $R_4$ represents a hydrogen atom, an aliphatic group, or an aryl group;

(ii) hydrolyzing the 1H-1,2,4-triazole derivative represented by formula (II) wherein $R_4$ is not a hydrogen atom to obtain a 1H-1,2,4-triazole derivative represented by formula (II) wherein $R_4$ is a hydrogen atom; and
(iii) reacting the 1H-1,2,4-triazole derivative represented by formula (II) wherein $R_4$ is a hydrogen atom with an acid halide represented by Y-X' in the presence of a base to obtain the 1H-pyrrolo[1,2-b][1,2,4]triazole derivative represented by formula (III-a):

wherein Y represents -C(=O)-$R_5$, -$SO_2R_6$ or -P(=O)($R_7$)($R_8$), in which $R_5$, $R_7$ and $R_8$ each represents a hydrogen atom, an aliphatic group which may be substituted, an aryl group which may be substituted, an aliphatic oxy group which may be substituted, an aryloxy group which may be substituted, or a group represents by -N(Ra')Ra, and $R_7$ and $R_8$ may be bonded to each other to form a ring; $R_6$ represents an aliphatic group which may be substituted, an aryl group which may be substituted, or a group represented by -N(Ra')Ra; and wherein Ra and Ra' each represents a hydrogen atom, an aliphatic group which may be substituted, or an aryl group which may be substituted, and Ra and Ra' may be bonded to each other to form a ring; and X' represents a halogen atom.

## Patentansprüche

1. 1H-1,2,4-Triazolderivat, dargestellt durch die Formel (I)

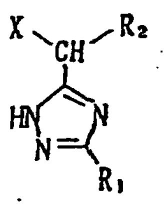

(I)

worin $R_1$ eine aliphatische Gruppe, die substituiert sein kann, oder eine Arylgruppe, die substituiert sein kann, ist; $R_2$ ist eine elektronenabziehende Gruppe mit einer Hammett-Substituentenkonstante $\sigma_p$ im Bereich von 0,2 bis 1,0; und X ist ein Halogenatom.

2. 1H-1,2,4-Triazolderivat nach Anspruch 1, wobei die aliphatische Gruppe, die durch $R_1$ dargestellt ist, eine geradkettige oder verzweigte Alkylgruppe mit 1 bis 36 Kohlenstoffatomen, eine Aralkylgruppe mit bis zu 36 Kohlenstoffatomen, eine Alkenylgruppe mit bis zu 36 Kohlenstoffatomen, eine Alkinylgruppe mit bis zu 36 Kohlenstoffatomen, eine Cycloalkylgruppe mit bis zu 36 Kohlenstoffatomen oder eine Cycloalkenylgruppe mit bis zu 36 Kohlenstoffatomen ist.

3. 1H-1,2,4-Triazolderivat nach Anspruch 1 oder 2, wobei $R_2$ eine Acylgruppe, eine Acyloxygruppe, eine Carbamoylgruppe, eine aliphatische Oxycarbonylgruppe, eine Aryloxycarbonylgruppe, eine Cyanogruppe, eine Nitrogruppe, eine Dialkylphosphonogruppe, eine Diarylphosphonogruppe, eine Diarylphosphinylgruppe, eine Alkylsulfinylgruppe, eine Arylsulfinylgruppe, eine Alkylsulfonylgruppe, eine Arylsulfonylgruppe, eine Sulfonyloxygruppe, eine Acylthiogruppe, eine Sulfamoylgruppe, eine Thiocyanatgruppe, eine Thiocarbonylgruppe, eine Alkylgruppe, die mit mindestens zwei Halogenatomen substituiert ist, eine Alkoxygruppe, die mit mindestens zwei Halogenatomen substituiert ist, eine Aryloxygruppe, die mit mindestens zwei Halogenatomen substituiert ist, eine Alkylaminogruppe, die mit mindestens zwei Halogenatomen substituiert ist, eine Alkylthiogruppe, die mit mindestens zwei Halogenatomen substituiert ist, eine Arylgruppe, die mit einer anderen elektronenabziehenden Gruppe mit einem $\sigma_p$-Wert von 0,20 oder darüber substituiert ist, eine heterocyclische Gruppe, ein Chloratom, ein Bromatom, eine Azogruppe oder eine Selenocyanatgruppe ist.

4. 1H-1,2,4-Triazolderivat nach Anspruch 1 oder 2, wobei $R_2$ eine aliphatische Oxycarbonylgruppe, die substituiert sein kann, ist.

5. 1H-1,2,4-Triazolderivat nach Anspruch 1 oder 2, wobei $R_2$ eine aliphatische Oxycarbonylgruppe ist, dargestellt durch die Formel (V)

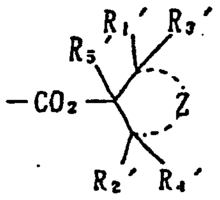

(V)

worin $R_1'$ und $R_2'$ jeweils eine aliphatische Gruppe bedeuten; $R_3'$, $R_4'$ und $R_5'$ bedeuten jeweils ein Wasserstoffatom oder eine aliphatische Gruppe; und Z ist eine Gruppe von Nichtmetallatomen, die erforderlich ist, um einen 5- bis 8-gliedrigen Ring zu bilden.

**6.** 1H-1,2,4-Triazolderivat nach einem der Ansprüche 1 bis 5, wobei X ein Bromatom oder ein Chloratom ist.

**7.** 1H-1,2,4-Triazolderivat nach Anspruch 1, dargestellt durch die Formel (I')

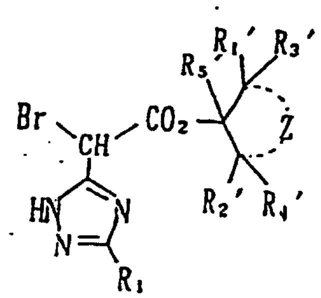

(I')

worin $R_1$ eine aliphatische Gruppe, die substituiert sein kann, oder eine Arylgruppe, die substituiert sein kann, ist; $R_1'$ und $R_2'$ bedeuten jeweils eine aliphatische Gruppe; $R_3'$, $R_4'$ und $R_5'$ bedeuten jeweils ein Wasserstoffatom oder eine aliphatische Gruppe; und Z ist eine Gruppe von Nichtmetallatomen, die erforderlich ist, um einen 5- bis 8-gliedrigen Ring zu bilden.

**8.** 1H-1,2,4-Triazolderivat, dargestellt durch die Formel (II)

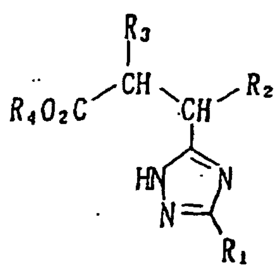

(II)

worin $R_1$ eine aliphatische Gruppe, die substituiert sein kann, oder eine Arylgruppe, die substituiert sein kann, ist; $R_2$ und $R_3$ bedeuten jeweils eine elektronenabziehende Gruppe mit einer Hammett-Substituentenkonstante $\sigma_p$ im Bereich von 0,2 bis 1,0; und $R_4$ ist ein Wasserstoffatom, eine aliphatische Gruppe oder eine Arylgruppe.

**9.** 1H-1,2,4-Triazolderivat nach Anspruch 8, wobei die aliphatische Gruppe, die durch $R_1$ dargestellt ist, eine geradkettige oder verzweigte Alkylgruppe mit 1 bis 36 Kohlenstoffatomen, eine Aralkylgruppe mit bis zu 36 Kohlenstoffatomen, eine Alkenylgruppe mit bis zu 36 Kohlenstoffatomen, eine Alkinylgruppe mit bis zu 36 Kohlenstoffatomen, eine Cycloalkylgruppe mit bis zu 36 Kohlenstoffatomen oder eine Cycloalkenylgruppe mit bis zu 36 Kohlenstoffatomen ist.

**10.** 1H-1,2,4-Triazolderivat nach Anspruch 8 oder 9, wobei $R_2$ und $R_3$ jeweils eine Acylgruppe, eine Acyloxygruppe, eine Carbamoylgruppe, eine aliphatische Oxycarbonylgruppe, eine Aryloxycarbonylgruppe, eine Cyanogruppe, eine Nitrogruppe, eine Dialkylphosphonogruppe, eine Diarylphosphonogruppe, eine Diarylphosphinylgruppe, eine Alkylsulfinylgruppe, eine Arylsulfinylgruppe, eine Alkylsulfonylgruppe, eine Arylsulfonylgruppe, eine Sulfonyloxygruppe, eine Acylthiogruppe, eine Sulfamoylgruppe, eine Thiocyanatgruppe, eine Thiocarbonylgruppe, eine Alkylgruppe, die mit mindestens zwei Halogenatomen substituiert ist, eine Alkoxygruppe, die mit mindestens zwei Halogenatomen substituiert ist, eine Aryloxygruppe, die mit mindestens zwei Halogenatomen substituiert ist, eine Alkylaminogruppe, die mit mindestens zwei Halogenatomen substituiert ist, eine Alkylthiogruppe, die mit mindestens zwei Halogenatomen substituiert ist, eine Arylgruppe, die mit einer anderen elektronenabziehenden Gruppe mit einem $\sigma_p$-Wert von 0,20 oder darüber substituiert ist, eine heterocyclische Gruppe, ein Chloratom, ein Bromatom, eine Azogruppe oder eine Selenocyanatgruppe bedeuten.

**11.** 1H-1,2,4-Triazolderivat nach Anspruch 8 oder 9, wobei $R_2$ eine aliphatische Oxycarbonylgruppe, die substituiert sein kann, ist und $R_3$ ist eine Cyanogruppe.

**12.** 1H-1,2,4-Triazolderivat nach Anspruch 8 oder 9, wobei $R_2$ eine aliphatische Oxycarbonylgruppe ist, dargestellt durch die Formel (V)

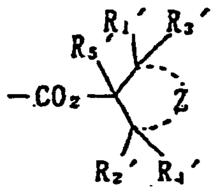

(V) ,

worin $R_1$' und $R_2$' jeweils eine aliphatische Gruppe bedeuten; $R_3$', $R_4$' und $R_5$' bedeuten jeweils ein Wasserstoffatom oder eine aliphatische Gruppe; und Z ist eine Gruppe von Nichtmetallatomen, die erforderlich ist, um einen 5- bis 8-gliedrigen Ring zu bilden.

**13.** 1H-1,2,4-Triazolderivat nach einem der Ansprüche 8 bis 12, wobei $R_3$ eine Cyanogruppe, eine aliphatische Oxycarbonylgruppe, eine Dialkylphosphonogruppe, eine Alkylsulfonylgruppe, eine Arylsulfonylgruppe oder eine Fluoralkylgruppe ist.

**14.** 1H-1,2,4-Triazolderivat nach einem der Ansprüche 8 bis 13, wobei $R_4$ ein Wasserstoffatom oder eine Alkylgruppe ist.

**15.** 1H-1,2,4-Triazolderivat nach Anspruch 8, dargestellt durch die Formel (II')

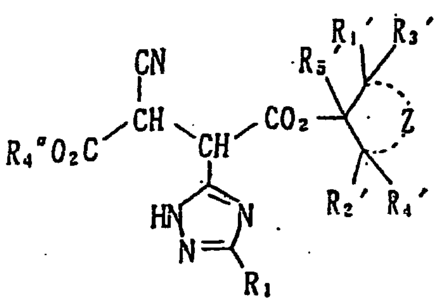

(II')

worin $R_1$ eine aliphatische Gruppe, die substituiert sein kann, oder eine Arylgruppe, die substituiert sein kann, ist; $R_1$' und $R_2$' bedeuten jeweils eine aliphatische Gruppe; $R_3$', $R_4$' und $R_5$' bedeuten jeweils ein Wasserstoffatom

oder eine aliphatische Gruppe; $R_4$" ist ein Wasserstoffatom oder eine Alkylgruppe mit 1 bis 7 Kohlenstoffatomen; und Z ist eine Gruppe von Nichtmetallatomen, die erforderlich ist, um einen 5- bis 8-gliedrigen Ring zu bilden.

**16.** Verfahren zur Herstellung des 1H-1,2,4-Triazolderivats nach Anspruch 8, umfassend:

(i) das Umsetzen eines 1H-1,2,4-Triazolderivats, dargestellt durch die Formel (I), mit einer aktiven Methylenverbindung, dargestellt durch die Formel $R_3$-$CH_2$-$CO_2R_4$''', in Gegenwart einer Base, um ein 1H-1,2,4-Triazolderivat, dargestellt durch die Formel (II), worin $R_4$ kein Wasserstoffatom ist, zu erhalten:

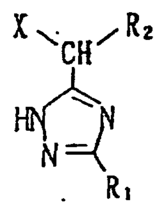

(I)

worin $R_1$ eine aliphatische Gruppe, die substituiert sein kann, oder eine Arylgruppe, die substituiert sein kann, ist; $R_2$ ist eine elektronenabziehende Gruppe mit einer Hammett-Substituentenkonstante $\sigma_p$ im Bereich von 0,2 bis 1,0; und X ist ein Halogenatom, und wobei $R_3$ eine Cyanogruppe, eine aliphatische Oxycarbonylgruppe, eine Dialkylphosphonogruppe, eine Alkylsulfonylgruppe, eine Arylsulfonylgruppe oder eine Fluoralkylgruppe ist; und $R_4$''' ist eine aliphatische Gruppe oder eine Arylgruppe; oder

(ii) das wahlweise Hydrolysieren des 1H-1,2,4-Triazolderivats, dargestellt durch die Formel (II), worin $R_4$ kein Wasserstoffatom ist, um ein 1H-1,2,4-Triazolderivat, dargestellt durch die Formel (II), worin $R_4$ ein Wasserstoffatom ist, zu erhalten.

**17.** 1H-Pyrrolo[1,2-b][1,2,4]triazolderivat, dargestellt durch die Formel (III)

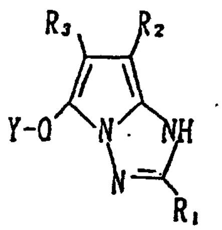

(III)

worin $R_1$ eine aliphatische Gruppe, die substituiert sein kann, oder eine Arylgruppe, die substituiert sein kann, ist; $R_2$ und $R_3$ bedeuten jeweils eine elektronenabziehende Gruppe mit einer Hammett-Substituentenkonstante $\sigma_p$ im Bereich von 0,2 bis 1,0; Y ist die Gruppe -C(=O)-N(Ra')Ra, worin Ra und Ra' jeweils ein Wasserstoffatom, eine aliphatische Gruppe, die substituiert sein kann, oder eine Arylgruppe, die substituiert sein kann, bedeuten, und Ra und Ra' können miteinander verbunden sein, um einen Ring zu bilden.

**18.** 1H-Pyrrolo[1,2-b][1,2,4]triazolderivat nach Anspruch 17, wobei die aliphatische Gruppe, die durch R, dargestellt ist, eine geradkettige oder verzweigte Alkylgruppe mit 1 bis 36 Kohlenstoffatomen, eine Aralkylgruppe mit bis zu 36 Kohlenstoffatomen, eine Alkenylgruppe mit bis zu 36 Kohlenstoffatomen, eine Alkinylgruppe mit bis zu 36 Kohlenstoffatomen, eine Cycloalkylgruppe mit bis zu 36 Kohlenstoffatomen oder eine Cycloalkenylgruppe mit bis zu 36 Kohlenstoffatomen ist.

**19.** 1H-Pyrrolo[1,2-b][1,2,4]triazolderivat nach Anspruch 17 oder 18, wobei $R_2$ und $R_3$ jeweils eine Acylgruppe, eine Acyloxygruppe, eine Carbamoylgruppe, eine aliphatische Oxycarbonylgruppe, eine Aryloxycarbonylgruppe, eine Cyanogruppe, eine Nitrogruppe, eine Dialkylphosphonogruppe, eine Diarylphosphonogruppe, eine Diarylphosphinylgruppe, eine Alkylsulfinylgruppe, eine Arylsulfinylgruppe, eine Alkylsulfonylgruppe, eine Arylsulfonylgruppe, eine Sulfonyloxygruppe, eine Acylthiogruppe, eine Sulfamoylgruppe, eine Thiocyanatgruppe, eine Thiocarbonylgruppe, eine Alkylgruppe, die mit mindestens zwei Halogenatomen substituiert ist, eine Alkoxygruppe, die mit mindestens zwei Halogenatomen substituiert ist, eine Aryloxygruppe, die mit mindestens zwei Halogenatomen substituiert ist, eine Alkylaminogruppe, die mit mindestens zwei Halogenatomen substituiert ist, eine Alkylthiogruppe, die mit mindestens zwei Halogenatomen substituiert ist, eine Arylgruppe, die mit einer anderen elektronenabziehenden Gruppe mit einem $\sigma_p$-Wert von 0,20 oder darüber substituiert ist, eine heterocyclische Gruppe, ein Chloratom, ein Bromatom, eine Azogruppe oder eine Selenocyanatgruppe bedeuten.

**20.** 1H-Pyrrolo[1,2-b][1,2,4]triazolderivat nach Anspruch 17 oder 18, wobei $R_2$ eine aliphatische Oxycarbonylgruppe, die substituiert sein kann, ist und $R_3$ ist eine Cyanogruppe.

**21.** 1H-Pyrrolo[1,2-b][1,2,4]triazolderivat nach Anspruch 17 oder 18, wobei $R_2$ eine aliphatische Oxycarbonylgruppe ist, dargestellt durch die Formel (V)

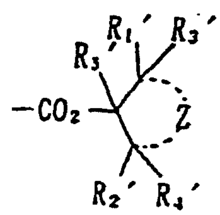

(V)

worin $R_1$' und $R_2$' jeweils eine aliphatische Gruppe bedeuten; $R_3$', $R_4$' und $R_5$' bedeuten jeweils ein Wasserstoffatom oder eine aliphatische Gruppe; und Z ist eine Gruppe von Nichtmetallatomen, die erforderlich ist, um einen 5- bis 8-gliedrigen Ring zu bilden.

**22.** 1H-Pyrrolo[1,2-b][1,2,4]triazolderivat nach einem der Ansprüche 17 bis 21, wobei $R_3$ eine Cyanogruppe, eine aliphatische Oxycarbonylgruppe, eine Dialkylphosphonogruppe, eine Alkylsulfonylgruppe, eine Arylsulfonylgruppe oder eine Fluoralkylgruppe ist.

**23.** 1H-Pyrrolo[1,2-b][1,2,4]triazolderivat nach Anspruch 17 oder 18, dargestellt durch die Formel (III')

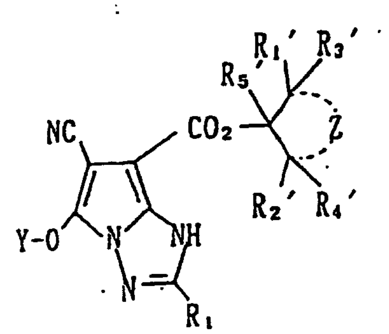

(III')

worin $R_1$ eine aliphatische Gruppe, die substituiert sein kann, oder eine Arylgruppe, die substituiert sein kann, ist; $R_1$' und $R_2$' bedeuten jeweils eine aliphatische Gruppe; $R_3$', $R_4$' und $R_5$' bedeuten jeweils ein Wasserstoffatom oder eine aliphatische Gruppe; Y ist die Gruppe -C(=O)-N(Ra')Ra, worin Ra und Ra' jeweils ein Wasserstoffatom, eine aliphatische Gruppe, die substituiert sein kann, oder eine Arylgruppe, die substituiert sein kann, bedeuten, und Ra und Ra' können miteinander verbunden sein, um einen Ring zu bilden; und Z ist eine Gruppe von Nichtmetallatomen, die erforderlich ist, um einen 5- bis 8-gliedrigen Ring zu bilden.

**24.** Verfahren zur Herstellung eines 1H-Pyrrolo[1,2-b][1,2,4]triazolderivats, dargestellt durch die Formel (III-a)

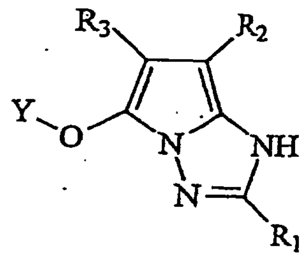

(III-a)

umfassend das Umsetzen eines 1H-1,2,4-Triazolderivats, dargestellt durch die Formel (II''), mit einem Säurehalogenid, dargestellt durch die Formel Y-X', in Gegenwart einer Base:

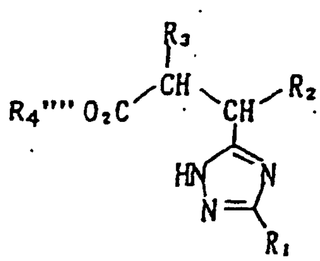

(II'')

worin $R_1$ eine aliphatische Gruppe, die substituiert sein kann, oder eine Arylgruppe, die substituiert sein kann, ist; $R_2$ und $R_3$ bedeuten jeweils eine elektronenabziehende Gruppe mit einer Hammett-Substituentenkonstante $\sigma_p$ im Bereich von 0,2 bis 1,0; und $R_4''''$ ist ein Wasserstoffatom; und Y ist die Gruppe -C(=O)-$R_5$, -$SO_2R_6$ oder - P(=O)($R_7$)($R_8$), worin $R_5$, $R_7$ und $R_8$ jeweils ein Wasserstoffatom, eine aliphatische Gruppe, die substituiert sein kann, eine Arylgruppe, die substituiert sein kann, eine aliphatische Oxygruppe, die substituiert sein kann, eine Aryloxygruppe, die substituiert sein kann, oder eine Gruppe, dargestellt durch die Formel -N(Ra')Ra, bedeuten, und $R_7$ und $R_8$ können miteinander verbunden sein, um einen Ring zu bilden; $R_6$ ist eine aliphatische Gruppe, die substituiert sein kann, eine Arylgruppe, die substituiert sein kann, oder eine Gruppe, dargestellt durch die Formel -N (Ra')Ra; und wobei Ra und Ra' jeweils ein Wasserstoffatom, eine aliphatische Gruppe, die substituiert sein kann, oder eine Arylgruppe, die substituiert sein kann, bedeuten, und Ra und Ra' können miteinander verbunden sein, um einen Ring zu bilden; und X ist ein Halogenatom.

**25.** Verfahren zur Herstellung eines 1H-Pyrrolo[1,2-b][1,2,4]triazolderivats, dargestellt durch die Formel (III-a)

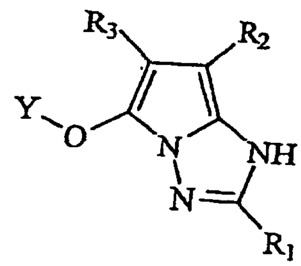

(III-a)

umfassend:

(i) das Umsetzen eines 1 H-1,2,4-Triazolderivats, dargestellt durch die Formel (I), mit einer aktiven Methylenverbindung, dargestellt durch die Formel $R_3$-$CH_2$-$CO_2R_4'''$, in Gegenwart einer Base, um ein 1H-1,2,4-Triazolderivat, dargestellt durch die Formel (II), worin $R_4$ kein Wasserstoffatom ist, zu erhalten:

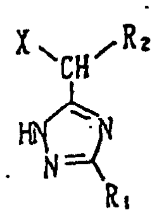

(I)

worin $R_1$ eine aliphatische Gruppe, die substituiert sein kann, oder eine Arylgruppe, die substituiert sein kann, ist; $R_2$ ist eine elektronenabziehende Gruppe mit einer Hammett-Substituentenkonstante $\sigma_p$ im Bereich von 0,2 bis 1,0; und X ist ein Halogenatom, und wobei $R_3$ eine Cyanogruppe, eine aliphatische Oxycarbonylgruppe, eine Dialkylphosphonogruppe, eine Alkylsulfonylgruppe, eine Arylsulfonylgruppe oder eine Fluoralkylgruppe ist; und $R_4'''$ ist eine aliphatische Gruppe oder eine Arylgruppe;

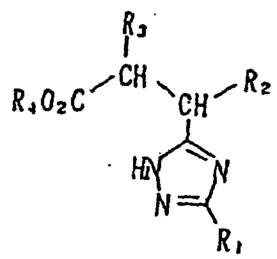

(II)

worin $R_1$ eine aliphatische Gruppe, die substituiert sein kann, oder eine Arylgruppe, die substituiert sein kann, ist; $R_2$ und $R_3$ bedeuten jeweils eine elektronenabziehende Gruppe mit einer Hammett-Substituentenkonstante $\sigma_p$ im Bereich von 0,2 bis 1,0; und $R_4$ ist ein Wasserstoffatom, eine aliphatische Gruppe oder eine Arylgruppe;

(ii) das Hydrolysieren des 1H-1,2,4-Triazolderivats, dargestellt durch die Formel (II), worin $R_4$ kein Wasserstoffatom ist, um ein 1H-1,2,4-Triazolderivat, dargestellt durch die Formel (II), worin $R_4$ ein Wasserstoffatom ist, zu erhalten; und

(iii) das Umsetzen des 1H-1,2,4-Triazolderivats, dargestellt durch die Formel (II), worin $R_4$ ein Wasserstoffatom ist, mit einem Säurehalogenid, dargestellt durch die Formel Y-X', in Gegenwart einer Base, um das 1H-Pyrrolo [1,2-b][1,2,4]triazolderivat, dargestellt durch die Formel (III-a), zu erhalten: worin Y die Gruppe $-C(=O)-R_5$, $-SO_2R_6$ oder $-P(=O)(R_7)(R_8)$ ist, worin $R_5$, $R_7$ und $R_8$ jeweils ein Wasserstoffatom, eine aliphatische Gruppe, die substituiert sein kann, eine Arylgruppe, die substituiert sein kann, eine aliphatische Oxygruppe, die substituiert sein kann, eine Aryloxygruppe, die substituiert sein kann, oder eine Gruppe, dargestellt durch die Formel -N(Ra')Ra, bedeuten, und $R_7$ und $R_8$ können miteinander verbunden sein, um einen Ring zu bilden; $R_6$ ist eine aliphatische Gruppe, die substituiert sein kann, eine Arylgruppe, die substituiert sein kann, oder eine Gruppe, dargestellt durch die Formel -N(Ra')Ra; und wobei Ra und Ra' jeweils ein Wasserstoffatom, eine aliphatische Gruppe, die substituiert sein kann, oder eine Arylgruppe, die substituiert sein kann, bedeuten, und Ra und Ra' können miteinander verbunden sein, um einen Ring zu bilden; und X' ist ein Halogenatom.

## Revendications

**1.** Dérivé du 1H-1,2,4-triazole représenté par la formule (I)

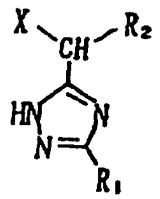

(I)

dans laquelle $R_1$ représente un groupe aliphatique qui peut être substitué ou un groupe aryle qui peut être substitué ; $R_2$ représente un groupe de retrait d'électrons ayant une constante de substituant de Hammett $\sigma_p$ de 0,2 à 1,0 ; et X représente un atome d'halogène.

2. Dérivé du 1H-1,2,4-triazole selon la revendication 1, dans lequel le groupe aliphatique représenté par $R_1$ représente un groupe alkyle linéaire ou ramifié ayant de 1 à 36 atomes de carbone, un groupe aralkyle ayant jusqu'à 36 atomes de carbone, un groupe alkényle ayant jusqu'à 36 atomes de carbone, un groupe alkynyle ayant jusqu'à 36 atomes de carbone, un groupe cycloalkyle ayant jusqu'à 36 atomes de carbone, ou un groupe cycloalkényle ayant jusqu'à 36 atomes de carbone.

3. Dérivé du 1H-1,2,4-triazole selon la revendication 1 ou 2, dans lequel $R_2$ représente un groupe acyle, un groupe acyloxy, un groupe carbamoyle, un groupe oxycarbonyle aliphatique, un groupe aryloxycarbonyle, un groupe cyano, un groupe nitro, un groupe dialkylphosphono, un groupe diarylphosphono, un groupe diarylphosphinyle, un groupe alkylsulfinyle, un groupe arylsulfinyle, un groupe alkylsulfonyle, un groupe arylsulfonyle, un groupe sulfonyloxy, un groupe acylthio, un groupe sulfamoyle, un groupe thiocyanate, un groupe thiocarbonyle, un groupe alkyle substitué par au moins deux atomes d'halogène, un groupe alkoxy substitué par au moins deux atomes d'halogène, un groupe aryloxy substitué par au moins deux atomes d'halogène, un groupe alkylamino substitué par au moins deux atomes d'halogène, un groupe alkylthio substitué par au moins deux atomes d'halogène, un groupe aryle substitué par un autre groupe de retrait d'électrons ayant une valeur $\sigma_p$ de 0,20 ou supérieure, un groupe hétérocyclique, un atome de chlore, un atome de brome, un groupe azo, ou un groupe sélénocyanate.

4. Dérivé du 1H-1,2,4-triazole selon la revendication 1 ou 2, dans lequel $R_2$ est un groupe oxycarbonyle aliphatique qui peut être substitué.

5. Dérivé du 1H-1,2,4-triazole selon la revendication 1 ou 2, dans lequel $R_2$ est un groupe oxycarbonyle aliphatique représenté par la formule (V)

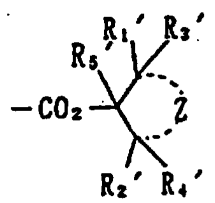

(V)

dans laquelle $R_1'$ et $R_2'$ représentent chacun un groupe aliphatique ; $R_3'$, $R_4'$ et $R_5'$ représentent chacun un atome d'hydrogène ou un groupe aliphatique ; et Z représente un groupe d'atomes non métalliques nécessaires pour constituer un anneau de 5 à 8 membres.

6. Dérivé du 1H-1,2,4-triazole selon l'une quelconque des revendications 1 à 5, dans lequel X est un atome de brome ou un atome de chlore.

7. Dérivé du 1H-1,2,4-triazole selon la revendication 1, qui est représenté par la formule (I')

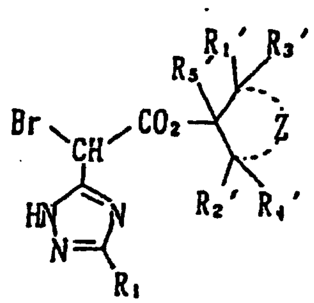

(I')

dans laquelle $R_1$ représente un groupe aliphatique qui peut être substitué ou un groupe aryle qui peut être substitué ; $R_1$' et $R_2$' représentent chacun un groupe aliphatique ; $R_3$', $R_4$' et $R_5$' représentent chacun un atome d'hydrogène ou un groupe aliphatique ; et Z représente un groupe d'atomes non métalliques nécessaires pour constituer un anneau de 5 à 8 membres.

**8.** Dérivé du 1H-1,2,4-triazole représenté par la formule (II)

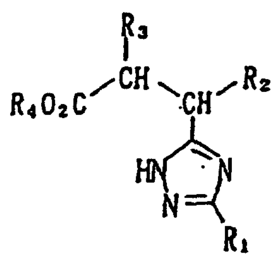

(II)

dans laquelle $R_1$ représente un groupe aliphatique qui peut être substitué ou un groupe aryle qui peut être substitué ; $R_2$ et $R_3$ représentent chacun un groupe de retrait d'électrons ayant une constante de substituant de Hammett $\sigma_p$ de 0,2 à 1,0 ; et $R_4$ représente un atome d'hydrogène, un groupe aliphatique, ou un groupe aryle.

**9.** Dérivé du 1H-1,2,4-triazole selon la revendication 8, dans lequel le groupe aliphatique représenté par $R_1$ représente un groupe alkyle linéaire ou ramifié ayant de 1 à 36 atomes de carbone, un groupe aralkyle ayant jusqu'à 36 atomes de carbone, un groupe alkényle ayant jusqu'à 36 atomes de carbone, un groupe alkynyle ayant jusqu'à 36 atomes de carbone, un groupe cycloalkyle ayant jusqu'à 36 atomes de carbone, ou un groupe cycloalkényle ayant jusqu'à 36 atomes de carbone.

**10.** Dérivé du 1H-1,2,4-triazole selon la revendication 8 ou 9, dans lequel $R_2$ et $R_3$ représentent chacun un groupe acyle, un groupe acyloxy, un groupe carbamoyle, un groupe oxycarbonyle aliphatique, un groupe aryloxycarbonyle, un groupe cyano, un groupe nitro, un groupe dialkylphosphono, un groupe diarylphosphono, un groupe diarylphosphinyle, un groupe alkylsulfinyle, un groupe arylsulfinyle, un groupe alkylsulfonyle, un groupe arylsulfonyle, un groupe sulfonyloxy, un groupe acylthio, un groupe sulfamoyle, un groupe thiocyanate, un groupe thiocarbonyle, un groupe alkyle substitué par au moins deux atomes d'halogène, un groupe alkoxy substitué par au moins deux atomes d'halogène, un groupe aryloxy substitué par au moins deux atomes d'halogène, un groupe alkylamino substitué par au moins deux atomes d'halogène, un groupe alkylthio substitué par au moins deux atomes d'halogène, un groupe aryle substitué par un autre groupe de retrait d'électrons ayant une valeur $\sigma_p$ de 0,20 ou supérieure, un groupe hétérocyclique, un atome de chlore, un atome de brome, un groupe azo, ou un groupe sélénocyanate.

**11.** Dérivé du 1H-1,2,4-triazole selon la revendication 8 ou 9, dans lequel $R_2$ est un groupe oxycarbonyle aliphatique qui peut être substitué et $R_3$ est un groupe cyano.

**12.** Dérivé du 1H-1,2,4-triazole selon la revendication 8 ou 9, dans lequel $R_2$ est un groupe oxycarbonyle aliphatique représenté par la formule (V)

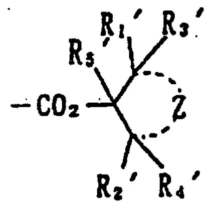

(V)

dans laquelle $R_1$' et $R_2$' représentent chacun un groupe aliphatique ; $R_3$', $R_4$' et $R_5$' représentent chacun un atome d'hydrogène ou un groupe aliphatique ; et Z représente un groupe d'atomes non métalliques nécessaires pour constituer un anneau de 5 à 8 membres.

**13.** Dérivé du 1H-1,2,4-triazole selon l'une quelconque des revendications 8 à 12, dans lequel $R_3$ représente un groupe cyano, un groupe oxycarbonyle aliphatique, un groupe dialkylphosphono, un groupe alkylsulfonyle, un groupe arylsulfonyle, ou un groupe fluoroalkyle.

**14.** Dérivé du 1H-1,2,4-triazole selon l'une quelconque des revendications 8 à 13, dans lequel $R_4$ est un atome d'hydrogène ou un groupe alkyle.

**15.** Dérivé du 1H-1,2,4-triazole selon la revendication 8, représenté par la formule (II')

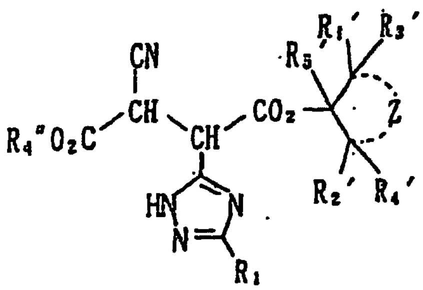

(II')

dans laquelle $R_1$ représente un groupe aliphatique qui peut être substitué ou un groupe aryle qui peut être substitué ; $R_1$' et $R_2$' représentent chacun un groupe aliphatique ; $R_3$', $R_4$' et $R_5$' représentent chacun un atome d'hydrogène ou un groupe aliphatique ; $R_4$" représente un atome d'hydrogène, ou un groupe alkyle ayant de 1 à 7 atomes de carbone ; et Z représente un groupe d'atomes non métalliques nécessaires pour constituer un anneau de 5 à 8 membres.

**16.** Procédé pour la production du dérivé du 1H-1,2,4-triazole selon la revendication 8, qui comprend :

(i) la mise en réaction d'un dérivé du 1H-1,2,4-triazole représenté par la formule (I) avec un composé de méthylène actif représenté par $R_3$-$CH_2$-$CO_2R_4$''' en présence d'une base pour obtenir un dérivé du 1H-1,2,4-triazole représenté par la formule (II) dans laquelle $R_4$ n'est pas un atome d'hydrogène :

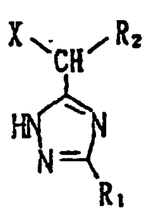

(I)

dans laquelle $R_1$ représente un groupe aliphatique qui peut être substitué ou un groupe aryle qui peut être substitué ; $R_2$ représente un groupe de retrait d'électrons ayant une constante de substituant de Hammett $\sigma_p$ de 0,2 à 1,0 ; et X représente un atome d'halogène, et dans laquelle $R_3$ représente un groupe cyano, un groupe oxycarbonyle aliphatique, un groupe dialkylphosphono, un groupe alkylsulfonyle, un groupe arylsulfonyle, ou un groupe fluoroalkyle ; et $R_4'''$ représente un groupe aliphatique ou un groupe aryle ; ou
(ii) l'hydrolisation facultative du dérivé du 1H-1,2,4-triazole représenté par la formule (II) dans laquelle $R_4$ n'est pas un atome d'hydrogène pour obtenir un dérivé du 1H-1,2,4-triazole représenté par la formule (II) dans laquelle $R_4$ est un atome d'hydrogène.

**17.** Dérivé du 1H-pyrrolo[1,2-b][1,2,4]triazole représenté par la formule (III)

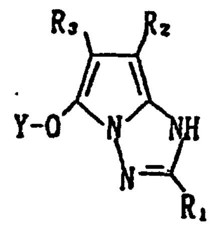

(III)

dans laquelle $R_1$ représente un groupe aliphatique qui peut être substitué ou un groupe aryle qui peut être substitué ; $R_2$ et $R_3$ représentent chacun un groupe de retrait d'électrons ayant une constante de substituant de Hammett $\sigma_p$ de 0,2 à 1,0 ; Y représente -C(=O)-N(Ra')Ra et où

Ra et Ra' représentent chacun un atome d'hydrogène, un groupe aliphatique qui peut être substitué, ou un groupe aryle qui peut être substitué, et Ra et Ra' peuvent être liés l'un à l'autre pour constituer un anneau.

**18.** Dérivé du 1H-pyrrolo[1,2-b][1,2,4]triazole selon la revendication 17, dans lequel le groupe aliphatique représente par $R_1$ représente un groupe alkyle linéaire ou ramifié ayant de 1 à 36 atomes de carbone, un groupe aralkyle ayant jusqu'à 36 atomes de carbone, un groupe alkényle ayant jusqu'à 36 atomes de carbone, un groupe alkynyle ayant jusqu'à 36 atomes de carbone, un groupe cycloalkyle ayant jusqu'à 36 atomes de carbone, ou un groupe cycloalkényle ayant jusqu'à 36 atomes de carbone.

**19.** Dérivé du 1H-pyrrolo[1,2-b][1,2,4]triazole selon la revendication 17 ou 18, dans lequel $R_2$ et $R_3$ représentent chacun un groupe acyle, un groupe acyloxy, un groupe carbamoyle, un groupe oxycarbonyle aliphatique, un groupe aryloxycarbonyle, un groupe cyano, un groupe nitro, un groupe dialkylphosphono, un groupe diarylphosphono, un groupe diarylphosphinyle, un groupe alkylsulfinyle, un groupe arylsulfinyle, un groupe alkylsulfonyle, un groupe arylsulfonyle, un groupe sulfonyloxy, un groupe acylthio, un groupe sulfamoyle, un groupe thiocyanate, un groupe thiocarbonyle, un groupe alkyle substitué par au moins deux atomes d'halogène, un groupe alkoxy substitué par au moins deux atomes d'halogène, un groupe aryloxy substitué par au moins deux atomes d'halogène, un groupe alkylamino substitué par au moins deux atomes d'halogène, un groupe alkylthio substitué par au moins deux atomes d'halogène, un groupe aryle substitué par un autre groupe de retrait d'électrons ayant une valeur $\sigma_p$ de 0,20 ou supérieure, un groupe hétérocyclique, un atome de chlore, un atome de brome, un groupe azo, ou un groupe sélénocyanate.

**20.** Dérivé du 1H-pyrrolo[1,2-b][1,2,4]triazole selon la revendication 17 ou 18, dans lequel $R_2$ est un groupe oxycarbonyle aliphatique qui peut être substitué et $R_3$ est un groupe cyano.

**21.** Dérivé du 1H-pyrrolo[1,2-b][1,2,4]triazole selon la revendication 17 ou 18, dans lequel $R_2$ est un groupe oxycarbonyle aliphatique représenté par la formule (V)

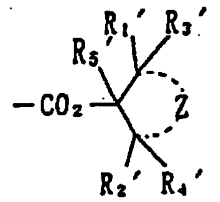

(V)

dans laquelle $R_1$' et $R_2$' représentent chacun un groupe aliphatique ; $R_3$', $R_4$' et $R_5$' représentent chacun un atome d'hydrogène ou un groupe aliphatique ; et Z représente un groupe d'atomes non métalliques nécessaires pour constituer un anneau de 5 à 8 membres.

**22.** Dérivé du 1H-pyrrolo[1,2-b][1,2,4]triazole selon l'une quelconque des revendications 17 à 21, dans lequel $R_3$ représente un groupe cyano, un groupe oxycarbonyle aliphatique, un groupe dialkylphosphono, un groupe alkylsulfonyle, un groupe arylsulfonyle, ou un groupe fluoroalkyle.

**23.** Dérivé du 1H-pyrrolo[1,2-b][1,2,4]triazole selon la revendication 17 ou 18, qui est représenté par la formule (III')

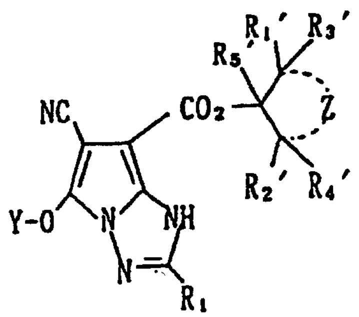

(III')

dans laquelle $R_1$ représente un groupe aliphatique qui peut être substitué ou un groupe aryle qui peut être substitué ; $R_1$' et $R_2$' représentent chacun un groupe aliphatique ; $R_3$', $R_4$' et $R_5$' représentent chacun un atome d'hydrogène ou un groupe aliphatique ;

Y représente -C(=O)-N(Ra')Ra et où

Ra et Ra' représentent chacun un atome d'hydrogène, un groupe aliphatique qui peut être substitué, ou un groupe aryle qui peut être substitué, et Ra et Ra' peuvent être liés l'un à l'autre pour constituer un anneau ; et

Z représente un groupe d'atomes non métalliques nécessaires pour constituer un anneau de 5 à 8 membres.

**24.** Procédé pour la production d'un dérivé du 1H-pyrrolo[1,2-b][1,2,4]triazole représenté par la formule (III-a)

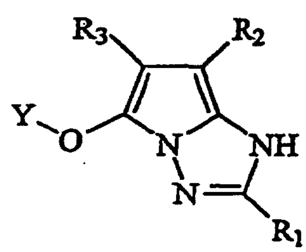

(III-a)

qui comprend la mise en réaction d'un dérivé du 1H-1,2,4-triazole représenté par la formule (II") avec un halogénure acide représenté par Y-X' en présence d'une base :

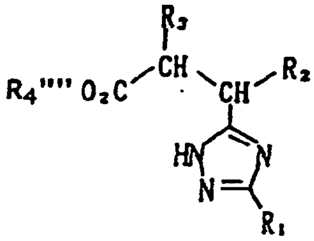

(II'')

dans laquelle $R_1$ représente un groupe aliphatique qui peut être substitué ou un groupe aryle qui peut être substitué ; $R_2$ et $R_3$ représentent chacun un groupe de retrait d'électrons ayant une constante de substituant de Hammett $\sigma_p$ de 0,2 à 1,0 ; et $R_4''''$ représente un atome d'hydrogène ; et

dans laquelle Y représente -C(=O)-$R_5$, -$SO_2R_6$ ou -P(=O)($R_7$)($R_8$), où $R_5$, $R_7$ et $R_8$ représentent chacun un atome d'hydrogène, un groupe aliphatique qui peut être substitué, un groupe aryle qui peut être substitué, un groupe oxy aliphatique qui peut être substitué, un groupe aryloxy qui peut être substitué, ou un groupe représenté par -N(Ra')Ra, et $R_7$ et $R_8$ peuvent être liés l'un à l'autre pour constituer un anneau ; $R_6$ représente un groupe aliphatique qui peut être substitué, un groupe aryle qui peut être substitué, ou un groupe représenté par -N(Ra') Ra ; et où Ra et Ra' représentent chacun un atome d'hydrogène, un groupe aliphatique qui peut être substitué, ou un groupe aryle qui peut être substitué, et Ra et Ra' peuvent être liés l'un à l'autre pour constituer un anneau ; et X' représente un atome d'halogène.

**25.** Procédé pour la production d'un dérivé du 1H-pyrrolo[1,2-b][1,2,4]triazole représenté par la formule (III-a)

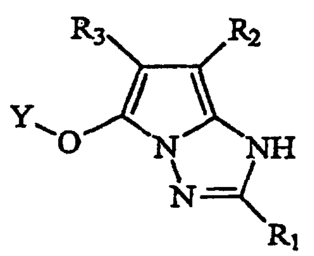

(III-a)

qui comprend :

(i) la mise en réaction d'un dérivé du 1H-1,2,4-triazole représenté par la formule (I) avec un composé de méthylène actif représenté par $R_3$-$CH_2$-$CO_2R_4'''$ en présence d'une base pour obtenir un dérivé du 1H-1,2,4-triazole représenté par la formule (II) dans laquelle $R_4$ n'est pas un atome d'hydrogène :

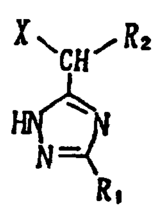

(I)

dans laquelle $R_1$ représente un groupe aliphatique qui peut être substitué ou un groupe aryle qui peut être substitué ; $R_2$ représente un groupe de retrait d'électrons ayant une constante de substituant de Hammett $\sigma_p$ de 0,2 à 1,0 ; et X représente un atome d'halogène, et dans laquelle $R_3$ représente un groupe cyano, un groupe oxycarbonyle aliphatique, un groupe dialkylphosphono, un groupe alkylsulfonyle, un groupe arylsulfonyle, ou un groupe fluoroalkyle ; et $R_4'''$ représente un groupe aliphatique ou un groupe aryle ;

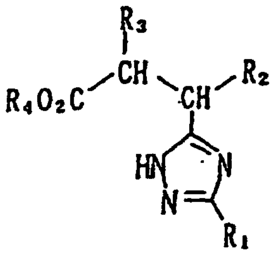

(II)

dans laquelle $R_1$ représente un groupe aliphatique qui peut être substitué ou un groupe aryle qui peut être substitué ; $R_2$ et $R_3$ représentent chacun un groupe de retrait d'électrons ayant une constante de substituant de Hammett $\sigma_p$ de 0,2 à 1,0 ; et $R_4$ représente un atome d'hydrogène, un groupe aliphatique ou un groupe aryle ;
(ii) l'hydrolisation du dérivé du 1H-1,2,4-triazole représenté par la formule (II) dans laquelle $R_4$ n'est pas un atome d'hydrogène pour obtenir un dérivé du 1H-1,2,4-triazole représenté par la formule (II) dans laquelle $R_4$ est un atome d'hydrogène ; et
(iii) la mise en réaction du dérivé du 1H-1,2,4-triazole représenté par la formule (II) dans laquelle $R_4$ est un atome d'hydrogène avec un halogénure acide représenté par Y-X' en présence d'une base pour obtenir le dérivé du 1H-pyrrolo[1,2-b][1,2,4]triazole représenté par la formule (III-a) ;

dans laquelle Y représente -C(=O)-$R_5$, -$SO_2R_6$ ou - P(=O)($R_7$)($R_8$), où $R_5$, $R_7$ et $R_8$ représentent chacun un atome d'hydrogène, un groupe aliphatique qui peut être substitué, un groupe aryle qui peut être substitué, un groupe oxy aliphatique qui peut être substitué, un groupe aryloxy qui peut être substitué, ou un groupe représenté par -N(Ra')Ra, et $R_7$ et $R_8$ peuvent être liés l'un à l'autre pour constituer un anneau ; $R_6$ représente un groupe aliphatique qui peut être substitué, un groupe aryle qui peut être substitué, ou un groupe représenté par -N(Ra') Ra ; et où Ra et Ra' représentent chacun un atome d'hydrogène, un groupe aliphatique qui peut être substitué, ou un groupe aryle qui peut être substitué, et Ra et Ra' peuvent être liés l'un à l'autre pour constituer un anneau ; et X' représente un atome d'halogène.